(19) Europäisches Patentamt
European Patent Office
Office européen des brevets

(11) Veröffentlichungsnummer: **0 350 445 A1**

(12) **EUROPÄISCHE PATENTANMELDUNG**

(21) Anmeldenummer: **89810481.5**

(22) Anmeldetag: **21.06.89**

(51) Int. Cl.5: **C 07 D 498/18**
A 61 K 31/495
//(C07D498/18,323:00,307:00,
263:00)

(30) Priorität: **30.06.88 CH 2500/88 02.03.89 CH 776/89**
**26.04.89 US 343980**

(43) Veröffentlichungstag der Anmeldung:
**10.01.90 Patentblatt 90/02**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Kump, Wilhelm, Dr.**
**Friedrich-Oser-Strasse 10**
**CH-4105 Biel-Benken (CH)**

**Chen, Jen**
**312 Clinton Avenue**
**+iddlesex New Jersey 08846 (US)**

Patentansprüche für folgende Vertragsstaaten: ES + GR

(54) **Substituierte Azacyclohexyl-Derivate.**

(57) Gegenstand der Erfindung sind neue substituierte Azacyclohexyl-Derivate der Formel

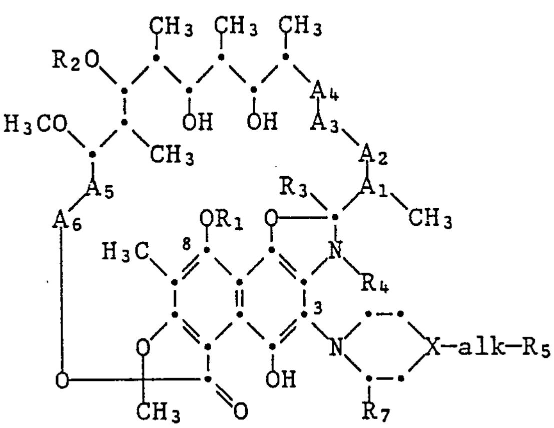

(I)

und ihre Salze, worin die Strukturelemente $-A_1-A_2-$, $-A_3-A_4-$ und $-A_5-A_6-$ jeweils Ethylen oder Vinylen oder die Elemente $-A_1-A_2-$ und $-A_3-A_4-$ jeweils Ethylen und $-A_5-A_6-$ Vinylen bedeuten, X für $>CR_6-$ oder $>N-$ und $R_6$ für Alkyl oder Wasserstoff steht, alk einen aliphatischen Kohlenwasserstoffrest oder eine Bindung bedeutet, $R_1$ Wasserstoff oder Acyl bedeutet, $R_2$ Wasserstoff oder Acetyl bedeutet, $R_3$ und $R_4$ gemeinsam für eine Bindung stehen oder jeweils Wasserstoff bedeuten, $R_5$ Wasserstoff, einen cycloaliphatischen Kohlenwasserstoffrest, Aryl oder Heteroaryl bedeutet und $R_7$ Wasserstoff oder Alkyl bedeutet, mit der Massgabe, dass wenn $-A_1-A_2-$, $-A_3-A_4-$ und $-A_5-A_6-$ jeweils Vinylen bedeuten, X für $>N-$ steht, $R_1$ Wasserstoff oder Trialkylacetyl bedeutet, $R_2$ Wasserstoff oder Acetyl bedeutet, $R_3$ und $R_4$ gemeinsam für eine Bindung stehen und alk Methylen bedeutet, $R_5$ von 2,6-Dimethyl-4-alkyl-phenyl verschieden ist, die als Arzneimittelwirkstoffe verwendet werden können, ihre Herstellung und Verwendung sowie pharmazeutische Präparate.

EP 0 350 445 A1

**Beschreibung**

**Substituierte Azacyclohexyl-Derivate**

Gegenstand der Erfindung sind neue substituierte Azacyclohexyl-Derivate von Rifamycinen der Formel

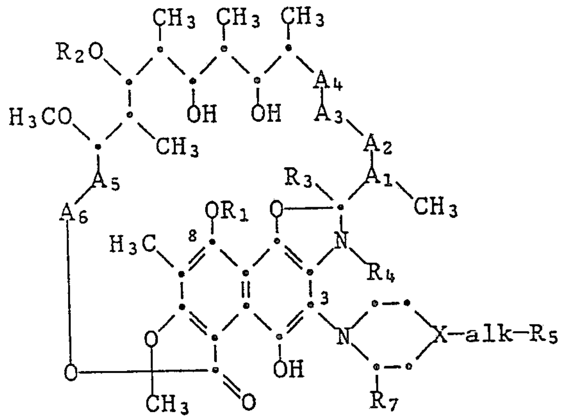

(I)

und ihre Salze, worin die Strukturelemente $-A_1-A_2-$, $-A_3-A_4-$ und $-A_5-A_6-$jeweils Ethylen oder Vinylen oder die Elemente $-A_1-A_2-$ und $-A_3-A_4-$jeweils Ethylen und $-A_5-A_6-$ Vinylen bedeuten, X für $> CR_6-$ oder $> N-$ und $R_6$ für Alkyl oder Wasserstoff steht, alk einen aliphatischen Kohlenwasserstoffrest oder eine Bindung bedeutet, $R_1$ Wasserstoff oder Acyl bedeutet, $R_2$ Wasserstoff oder Acetyl bedeutet, $R_3$ und $R_4$ gemeinsam für eine Bindung stehen oder jeweils Wasserstoff bedeuten, $R_5$ Wasserstoff, einen cycloaliphatischen Kohlenwasserstoffrest, Aryl oder Heteroaryl bedeutet und $R_7$ Wasserstoff oder Alkyl bedeutet, mit der Massgabe, dass wenn $-A_1-A_2-$, $-A_3-A_4-$ und $-A_5-A_6-$ jeweils Vinylen bedeuten, X für $> N-$steht, $R_1$ Wasserstoff oder Trialkylacetyl bedeutet, $R_2$ Wasserstoff oder Acetyl bedeutet, $R_3$ und $R_4$ gemeinsam für eine Bindung stehen und alk Methylen bedeutet, $R_5$ von 2,6-Dimethyl-4-alkyl-phenyl verschieden ist, ihre Herstellung und Verwendung sowie pharmazeutische Präparate und ihre Herstellung.

Die zugrundeliegende Numerierung des Ringsystems entspricht derjenigen, die z.B. im US Patent Nr. 4,005,077 angewandt wurde.

Die Verbindungen der Formel I besitzen mehrere Chiralistätszentren, dementsprechend umfasst die vorliegende Erfindung auch die entsprechenden optischen Isomeren, z.B. Diastereomeren.

Die Verbindungen der Formel I können als, insbesondere pharmazeutisch verwendbare, Salze vorliegen. Da die erfindungsgemässen Verbindungen mindestens ein basisches Zentrum aufweisen, können sie somit Säureadditionssalze bilden. Diese werden beispielsweise mit anorganischen Säuren, wie Mineralsäuren, z.B. Schwefelsäure, eine Phosphor- oder Halogenwasserstoffsäure, oder mit organischen Carbonsäuren, wie gegebenenfalls, z.B. durch Halogen, substituierte $C_1$-$C_4$-Alkancarbonsäuren, z.B. Essigsäure, wie gegebenenfalls ungesättigte Dicarbonsäuren, z.B. Oxal-, Malon-, Bernstein-, Malein-, Fumar-, Phthal-, oder Terephthalsäure, wie Hydroxycarbonsäuren, z.B. Ascorbin-, Glykol-, Milch-, Aepfel-, Wein- oder Citronensäure, wie Aminosäuren, z.B. Asparagin- oder Glutaminsäure, wie einer aromatischen Carbonsäure, z.B. Benzoesäure, oder mit organischen Sulfonsäuren, wie gegebenenfalls, z.B. durch Halogen, substituierte $C_1$-$C_4$-Alkan- oder Arylsulfonsäuren, z.B. Methan-, Brombenzol- oder Toluolsulfonsäure, gebildet. Entsprechende Säureadditionssalze können auch mit einem gegebenenfalls zusätzlich vorhandenen basischen Zentrum (z.B. $X = > N-$) gebildet werden. Ferner können die erfindungsgemässen Verbindungen mit einer aciden phenolischen Hydroxygruppe Salze mit Basen bilden, z.B. Alkalimetall-, wie Natrium- oder Kaliumsalze. Weiterhin können entsprechende innere Salze gebildet werden. Umfasst sind ferner für pharmazeutische Verwendungen nicht geeignete Salze, da diese beispielsweise für die Isolierung bzw. Reinigung erfindungsgemässer Verbindungen oder deren pharmazeutisch verwendbarer Salze eingesetzt werden können.

Ein aliphatischer Kohlenwasserstoffrest bedeutet insbesondere Alkylen, Alkenylen und Alkinylen, wobei in ungesättigten Resten die Mehrfachbindungen bevorzugt in höherer als der $\alpha$-Stellung zum Piperazinstickstoffatom ($X = > N-$) lokalisiert sind.

Acyl leitet sich z.B. von einer organischen Carbonsäure oder einer substituierten Kohlensäure ab. Derartige Reste sind beispielsweise gegebenenfalls, z.B. durch Halogen oder Aryl, substituiertes ($C_1$-$C_7$-)Alkanoyl, oder gegebenenfalls, z.B. durch Halogen, ($C_1$-$C_7$-)Alkyl, ($C_1$-$C_7$-)Alkoxy, Hydroxy, ($C_2$-$C_8$-)Alkanoyloxy, Trifluormethyl und/oder Nitro, substituiertes carbocyclisches Aroyl, wie Benzoyl oder Naphthoyl, oder heterocyclisches Aroyl, wie monocyclisches, 5- oder 6-gliedriges Monothia-, Monooxa- oder Monoazaaroyl, z.B. (2-)Thenoyl, (3-)Furoyl, Nicotinoyl oder Isonicotinoyl. Als von einer substituierten Kohlensäure abgeleiteter Acylrest kommt beispielsweise ($C_1$-$C_7$-)Alkoxycarbonyl oder gegebenenfalls, z.B. durch ($C_1$-$C_7$-)Alkyl, mono- oder disubstituiertes Aminocarbonyl in Betracht.

Ein cycloaliphatischer Kohlenwasserstoffrest bedeutet insbesondere Cycloalkyl, Cycloalkenyl oder

Cycloalkinyl, wobei derartige Reste zusätzlich eine oder mehrere, wie 2 oder 3, Alkylgruppen, insbesondere Niederalkyl, aufweisen können.

Aryl leiter sich z.B. von einem mono- oder polycyclischen, wie bicyclischen, C-Ringsystem ab, das mindestens einen aromatischen Ring aufweist, wie Phenyl, Biphenylyl, wie 2-, 3- oder insbesondere 4-Biphenylyl, oder Naphthyl, wie 1- oder 2-Naphthyl, und unsubstituiert oder ein- oder mehrfach, z.B. zwei-oder dreifach, beispielsweise durch Halogen, ($C_1$-$C_7$-)Alkyl, ($C_1$-$C_7$-)Alkoxy, Hydroxy, ($C_2$-$C_8$-)Alkanoyloxy, Trifluormethyl und/oder Nitro, substituiert ist.

Heteroaryl steht insbesondere für monocyclisches, 5- oder 6-gliedriges Monoaza-, Monooxa- und Monothiaaryl, wie Pyrrolyl, N-Alkylpyrrolyl, Pyridyl, N-Oxodo-pyridyl, Furyl und Thienyl, und ist unsubstituiert oder ein- oder mehrfach, wie zwei- oder dreifach, substituiert, beispielsweise wie für Aryl angegeben.

Die vor- und nachstehend verwendeten Allgemeindefinitionen haben, sofern nicht abweichend definiert, in erster Linie die folgenden Bedeutungen:

Alkyl bedeutet insbesondere $C_1$-$C_7$-Alkyl und ist z.B. Methyl, Ethyl, Propyl, Isopropyl, n-Butyl, Isobutyl, sek.-Butyl, tert.-Butyl und umfasst ferner entsprechende Pentyl-, Hexyl- und Heptylreste. Bevorzugt ist $C_1$-$C_4$-Alkyl.

Alkylen bedeutet beispielsweise $C_1$-$C_{12}$-Alkylen, insbesondere $C_1$-$C_7$-Alkylen, und ist geradkettig oder verzweigt und bedeutet z.B. Methylen, Ethylen, Propylen und Butylen sowie 1,2-Propylen, 2-Methyl-1,3-propylen oder 2,2-Dimethyl-1,3-propylen. Bevorzugt ist $C_1$-$C_4$-Alkylen, in erster Linie Methylen.

Alkenylen bedeutet insbesondere $C_3$-$C_7$-Alkenylen und ist geradkettig oder verzweigt und bedeutet z.B. 1,3-Prop-2-enylen, 1,4-But-2-, 1,4-But-3-enylen, 1,3-But-2-enylen, 2,4-But-3-enylen, 1,5-Pent-2-, -3-, -4-enylen, ferner entsprechende Hexenylen- und Heptenylenreste. Bevorzugt ist $C_3$-$C_5$-Alkenylen.

Alkinylen bedeutet insbesondere $C_3$-$C_7$-Alkinylen und ist geradkettig oder verzweigt und bedeutet z.B. 1,3-Prop-2-inylen, 1,4-But-2-, 1,4-But-3-inylen, 1,5-Pent-2-, -3- und -5-inylen, ferner entsprechende Hexinylen-und Heptinylenreste. Bevorzugt ist $C_3$-$C_5$-Alkinylen.

Halogen ist insbesondere Halogen mit Atomnummer bis und mit 35, wie Fluor, Chlor und Brom, ferner Iod.

Cycloalkyl bedeutet insbesondere $C_3$-$C_7$-Cycloalkyl und bedeutet z.B. Cyclopropyl, -butyl, -pentyl, -hexyl oder -heptyl. Bevorzugt ist Cyclopentyl und Cyclohexyl.

Cycloalkenyl bedeutet insbesondere $C_3$-$C_7$-Cycloalkenyl und enthält in erster Linie eine, ferner (ab $C_4$) zwei Doppelbindungen, und bedeutet z.B. Cycloprop-2-en-1-yl, Cyclobut-2-en-1-yl, Cyclobut-1,3-dien-1-yl, Cyclopent-2-en-1-yl, -3-en-1-yl, -2,4-dien-1-yl, Cyclohex-2-en-1-yl, -3-en-1-yl, -2,4-dien-1-yl, -1,3-dien-1-yl, -2,5-dien-1-yl, ferner einen entsprechenden Cycloheptenylrest.

Cycloalkinyl bedeutet insbesondere $C_3$-$C_7$-Cycloalkinyl und enthält in erster Linie eine Dreifachbindung und bedeutet z.B. Cyclopent-2-in-1-yl, Cyclobut-2-in-1-yl, Cyclopent-2-in-1-yl, -3-in-1-yl, Cyclohex-2-in-1-yl, -3-in-1-yl, ferner einen entsprechenden Heptinylrest.

Alkanoyl bedeutet insbesondere $C_2$-$C_8$-Alkanoyl und ist z.B. Acetyl, Propionyl, Butyryl, Isobutyryl oder Pivaloyl. Bevorzugt ist verzweigtes $C_3$-$C_6$-Alkanoyl, in erster Linie Pivaloyl.

Alkoxy bedeutet insbesondere $C_1$-$C_7$-Alkoxy und ist z.B. Methoxy, Ethoxy, Propyloxy, Isopropyloxy, n-Butyloxy, Isobutyloxy und tert.-Butyloxy. Bevorzugt ist $C_1$-$C_4$-Alkoxy.

In Alkanoyloxy hat Alkanoyl die vorstehend angegebenen Bedeutungen.

In Alkoxycarbonyl hat Alkoxy die vorstehend angegebenen Bedeutungen.

Pyrrolyl ist z.B. 1-, 2- und 3-Pyrrolyl, Pyridyl z.B. 2-, 3- und 4-Pyridyl, 1-Oxido-pyridyl z.B. 1-Oxido-2-, -3- und -4-pyridyl, Furyl z.B. 2- und 3-Furyl und Thienyl z.B. 2- und 3-Thienyl.

Von Derivaten, die sich beispielsweise vom Rifamycin-SV ableiten, ist bekannt, das sie ausgeprägte antibiotische Eigenschaften besitzen und beispielsweise zur Behandlung von Tuberkulose eingesetzt werden können. Es wurde nun festgestellt, dass die Verbindungen der Formel I und ihre pharmazeutisch verwendbaren Salze in den üblichen pharmakologischen Testmodellen zur Prüfung keine entsprechende antibiotische Aktivität zeigen.

Ueberraschenderweise dagegen besitzen sie jedoch eine signifikante lipidsenkende Wirkung, die in Tierversuchen, vorzugsweise an Säugetieren, z.B. an Ratten, nachgewiesen werden kann. So kann die Senkung von "very low density"-, "low-density"- bzw. "high-density"-Lipoproteinen (VLDL, LDL bzw. HDL) im Serum in zwei Testanordnungen, und zwar in genetisch hypercholesterolämischen männlichen Ratten (Anordnung A) und normolipämischen Ratten beiden Geschlechts (Anordnung B) gezeigt werden.

Albino-Ratten mit einem Körpergewicht von 180-240 g, die freien Zugang zu Standard-Rattenfutter und Trinkwasser haben, werden verwendet, und zwar in Anordnung A Sprague Dawley Abkömmlinge des Stammes Tif:RAI und in Anordnung B Tiere des Wistar-Stammes IVa-WI. Die Testverbindung wird in einer Polyethylenglykollösung (mittleres Molekulargewicht von 400) oral an Gruppen von 8 bis 10 Ratten täglich während 5 aufeinanderfolgenden Tagen verabreicht. Die Tiere werden zwei Stunden nach der letzten Gabe durch Ausblutenlassen vom Hals unter Anästhesie mit Ether getötet. Während 16 Stunden vor ihrem Tod erhalten die Tiere keine Nahrung mehr. Zum vereinigten Serum von 2 bis 3 Ratten fügt man eine 0,05%ige wässrige Ethylendiamintetraessigsäure-Lösung und eine 0,01%ige wässrige Thiomersal-Lösung. Die Serumlipoproteine werden unter Verwendung einer Ultrazentrifuge durch 24-stündiges Zentrifugieren bei 78 000 g, 78 000 bzw. 109 000 g in Salzlösungen mit den Dichten 1,006 bzw. 1,040 getrennt und auf Gehalt von Cholesterin und Triglyceriden enzymatisch mit Hilfe der z.B. von Sigma Chemical Co. (St.Louis, MO, USA) gelieferten Testsysteme analysiert.

Die Prüfung auf antibiotische Wirkung erfolgt z.B. einerseits in vitro durch die Bestimmung der mittleren

effektiven Konzentration $EC_{50}$ für die Hemmung der RNA-Polymerase von Escherichia coli, sowie der minimalen Hemmkonzentration MIC ("minimum inhibitory concentration") im konventionellen Plattentest, andererseits in vivo an infizierten Mäusen und Ratten durch die Bestimmung von $ED_{50}$ (effektive Dosis, die für 50 % der Versuchstiere lebenserhaltend ist). Als Mikroorganismen werden für den vorliegenden Zweck insbesondere Mycobacterium tuberculosis TB $H_{37}Rv$ und Staphylococcus aureus verwendet. Bei Verbindungen mit lipidsenkender Indikation wird eine antibiotische Wirksamkeit als nachteilig erachtet, da sie, insbesondere bei langfristiger Verabreichung, zur Bildung von gegen Antibiotika resistenten Stämmen von Mikroorganismen führen kann.

In den oben beschriebenen Testmethoden weisen die erfindungsgemässen Verbindungen bei wiederholter Verabreichung im Dosisbereich von etwa 0,1 bis etwa 50 mg/kg/Tag eine signifikante hypolipidämische Wirksamkeit auf; dagegen sind sie in den oben erwähnten Tests frei von nennenswerter antibiotischer Aktivität.

So kann z.B. gezeigt werden, dass, je nach Versuchsanordnung, die minimale effektive Dosis der erfindungsgemässen Verbindungen bei einmaliger Verabreichung bei etwa 0,1 bis etwa 10 mg/kg liegt, und durch eine wiederholte Verabreichung von täglich 30 mg/kg eine 50-70%ige Senkung der "LDL-Fraktion" erreicht werden kann. Dabei haben die Verbindungen praktisch keine antibiotische Wirksamkeit; eine $EC_{50}$ für die Inhibition der RNA-Polymerase wird mit 100 µg/ml noch nicht erreicht, und die MIC für verschiedene pathogene Stämme von Staphylococcus aureus liegt über 130 µg/ml. Solche Werte sind etwa 1000-fach höher als Konzentrationen, die für einen entsprechenden Effekt normalerweise erforderlich sind. Auch in vivo unter Verwendung von mit Staphylococcus aureus infizierten Mäusen erweist sich die Verbindung bei einer Einzeldosis von 200 mg/kg als antibiotisch unwirksam.

Insbesondere dank ihrer LDL-senkenden Wirkung können die erfindungsgemässen Verbindungen z.B. als Hypolipidämika zur Behandlung von Hyperlipidämien, hauptsächlich der Typen IIa und IIb, und Atherosclerose, z.B. bei Vorliegen von Hyperlipoproteinämie als Risikofaktor, verwendet werden.

Dementsprechend können die Verbindungen der Formel I und ihre pharmazeutisch verwendbaren Salze z.B. als Pharmazeutika, beispielsweise als Hypolipidämika zur Behandlung von Hyperlipidämien, hauptsächlich der Typen IIa und IIb, und von Arteriosklerose bei Vorliegen von Hyperlipoproteinamie als Risikofaktor, verwendet werden. Eine weiterer Gegenstand der Erfindung ist die Verwendung der erfindungsgemässen Verbindungen zur Herstellung von Arzneimitteln, insbesondere von Hypolipidämika und Antiarteriosklerotika, und zur therapeutischen und prophylaktischen Behandlung. Dabei ist auch die gewerbsmässige Herrichtung der Wirksubstanzen eingeschlossen.

Die Erfindung betrifft insbesondere Verbindungen der Formel I und ihre Salze, worin $-A_1-A_2-$, $-A_3-A_4-$, $-A_5-A_6-$, $R_2$, $R_3$ und $R_4$ die angegebenen Bedeutungen haben, X für >CH- oder >N- steht, alk Alkylen bedeutet, $R_1$ Wasserstoff oder Acyl bedeutet und $R_5$ Wasserstoff, Cycloalkyl oder Aryl bedeutet und $R_7$ Wasserstoff bedeutet.

Die Erfindung betrifft insbesondere Verbindungen der Formel I und ihre Salze, worin $-A_1-A_2-$, $-A_3-A_4-$, $-A_5-A_6-$, $R_2$, $R_3$ und $R_4$ die angegebenen Bedeutungen haben, X für >C($R_6$) oder >N- und $R_6$ für Wasserstoff oder Alkyl steht, alk Alkylen, Alkenylen oder Alkinylen bedeutet, wobei die Mehrfachbindung in höherer als der α-Stellung zum Piperazinstickstoff (X = >N-) lokalisiert ist, $R_1$ gegebenenfalls durch Halogen oder durch Phenyl substituiertes Alkanoyl, Benzoyl, Naphthoyl oder monocyclisches, 5- bis 6-gliedriges Monoaza-, Monooxa- oder Monothiaaroyl, Alkoxycarbonyl oder gegebenenfalls durch Alkyl mono- oder disubstituiertes Aminocarbonyl bedeutet, $R_5$ Wasserstoff, Cycloalkyl, Cycloalkenyl, Cycloalkinyl, Phenyl, Biphenylyl, Naphthyl, monocyclisches, 5- oder 6-gliedriges Monoazo-, Monooxa- oder Monothiaaryl bedeutet, und $R_7$ Wasserstoff oder Alkyl bedeutet, wobei die aromatischen Reste unabhängig voneinander jeweils unsubstituiert oder ein- oder mehrfach durch Halogen, Alkyl, Alkoxy, Hydroxy, Alkanoyloxy, Trifluormethyl und/oder Nitro substituiert sind.

Die Erfindung betrifft insbesondere Verbindungen der Formel I und ihre Salze, worin $-A_1-A_2-$, $-A_3-A_4-$, $-A_5-A_6-$, $R_2$, $R_3$ und $R_4$ die angegebenen Bedeutungen haben, X für >C($R_6$) oder >N- und $R_6$ für Wasserstoff oder $C_1$-$C_7$-Alkyl steht, alk $C_1$-$C_{12}$-Alkylen, $C_3$-$C_7$-Alkenylen oder $C_3$-$C_7$-Alkinyllen bedeutet, wobei die Mehrfachbindung in höherer als der α-Stellung zum Piperazinstickstoffatom lokalisiert ist, $R_1$ gegebenenfalls durch Halogen oder durch Phenyl substituiertes $C_2$-$C_8$-Alkanoyl, Benzoyl, Naphthoyl oder monocyclisches, 5- bis 6-gliedriges Monoaza-, Monooxa- oder Monothiaaroyl, $C_1$-$C_7$-Alkoxycarbonyl oder gegebenenfalls durch $C_1$-$C_7$-Alkyl mono-oder disubstituiertes Aminocarbonyl bedeutet, $R_5$ Wasserstoff, $C_3$-$C_7$-Cycloalkyl, $C_3$-$C_7$-Cycloalkenyl, $C_3$-$C_7$-Cycloalkinyl, Phenyl, Biphenylyl, Naphthyl, Pyrrolyl, N-$C_1$-$C_7$-Alkyl-pyrrolyl, Pyridyl, 1-Oxido-pyridyl, Furyl oder Thienyl bedeutet, und $R_7$ Wasserstoff oder $C_1$-$C_7$-Alkyl bedeutet, wobei die aromatischen Reste unabhängig voneinander jeweils unsubstituiert oder ein- oder mehrfach durch Halogen, $C_1$-$C_7$-Alkyl, $C_1$-$C_7$-Alkoxy, Hydroxy, $C_2$-$C_8$-Alkanoyloxy, Trifluormethyl und/oder Nitro substituiert sind.

Die Erfindung betrifft insbesondere Verbindungen der Formel I und ihre Salze, worin $-A_1-A_2-$, $A_3-A_4-$, $-A_5-A_6-$, $R_2$, $R_3$ und $R_4$ die angegebenen Bedeutungen haben, X für >CH- oder >N- steht, alk $C_1$-$C_7$-Alkylen bedeutet, $R_1$ gegebenenfalls durch Halogen oder durch gegebenenfalls Halogen, $C_1$-$C_7$-Alkyl, $C_1$-$C_7$-Alkoxy, Hydroxy, $C_2$-$C_8$-Alkanoyloxy, Trifluormethyl, und/oder Nitro aufweisendes Phenyl substituiertes $C_2$-$C_8$-Alkanoyl, gegebenenfalls durch Halogen, $C_1$-$C_7$-Alkyl, $C_1$-$C_7$-Alkoxy, Hydroxy, $C_2$-$C_8$-Alkanoyloxy, Trifluormethyl, und/oder Nitro substituiertes Benzoyl, Naphthoyl oder monocyclisches, 5- bis 6-gliedriges Monoaza-, Monooxa- oder Monothiaaroyl, $C_1$-$C_7$-Alkoxycarbonyl oder gegebenenfalls durch $C_1$-$C_7$-Alkyl mono- oder disubstituiertes Aminocarbonyl bedeutet, $R_5$ Wasserstoff, $C_3$-$C_7$-Cycloalkyl oder unsubstituiertes oder ein-

oder mehrfach durch Halogen, $C_1$-$C_7$-Alkyl, $C_1$-$C_7$-Alkoxy, Hydroxy, $C_2$-$C_8$-Alkanoyloxy, Trifluormethyl und/oder Nitro substituiertes Phenyl, Biphenylyl oder Naphthyl bedeutet und $R_7$ Wasserstoff ist.

Die Erfindung betrifft insbesondere Verbindungen der Formel I und ihre Salze, worin $-A_1-A_2-$, $-A_3-A_4-$, $-A_5-A_6-$, $R_2$, $R_3$ und $R_4$ die angegebenen Bedeutungen haben, X für $>C(R_6)$ oder $>N-$ und $R_6$ für Wasserstoff oder $C_1$-$C_7$-Akyl steht, alk $C_1$-$C_{12}$-Alkylen, wie $C_1$-$C_7$-Alkylen, $C_3$-$C_7$-Alkenylen oder $C_3$-$C_7$-Alkinylen bedeutet, wobei die Mehrfachbindung in höherer als der α-Stellung zum Piperazinstickstoffatom lokalisiert ist, $R_1$ Wasserstoff oder $C_3$-$C_6$-Alkanoyl bedeutet, $R_5$ Wasserstoff, $C_3$-$C_7$-Cycloalkyl, $C_3$-$C_7$-Cycloalkenyl oder unsubstituiertes oder durch Halogen, $C_1$-$C_7$-Alkyl, $C_1$-$C_7$-Alkoxy und/oder Trifluormethyl substituiertes Phenyl, Biphenylyl, Naphthyl, Thienyl, Furyl oder Pyridyl bedeutet und $R_7$ Wasserstoff oder $C_1$-$C_7$-Alkyl bedeutet.

Die Erfindung betrifft insbesondere Verbindungen der Formel I und ihre Salze, worin $-A_1-A_2-$, $-A_3-A_4-$, $-A_5-A_6-$, $R_3$ und $R_4$ die angegebenen Bedeutungen haben, X für $>CH$ oder $>N-$ steht, $R_1$ $C_3$-$C_6$-Alkanoyl, insbesondere Pivaloyl, bedeutet, $R_2$ Acetyl ist, einerseits alk $C_3$-$C_7$-Alkylen, wie 2-Methyl-1,3-propylen, bedeutet und $R_5$ Wasserstoff ist oder andererseits alk $C_1$-$C_4$-Alkylen, insbesondere Methylen, bedeutet, $R_5$ $C_3$-$C_6$-Cycloalkyl, wie Cyclohexyl, $C_3$-$C_6$-Cycloalkenyl, wie Cyclohex-3-en-1-yl, unsubstituiertes oder durch $C_1$-$C_4$-Alkyl, insbesondere Methyl, substituiertes Phenyl, wie 2,5-Dimethyl- oder 2,4,6-Trimethylphenyl, Biphenylyl, wie 4-Biphenylyl, Naphthyl, wie 2-Naphthyl, oder Thienyl, wie 2-Thienyl, bedeutet und $R_7$ Wasserstoff oder $C_1$-$C_4$-Alkyl, wie Methyl, bedeutet.

Die Erfindung betrifft insbesondere Verbindungen der Formel I und ihre Salze, worin $-A_2-$, $-A_3-A_4-$, $-A_5-A_6-$, $R_3$ und $R_4$ die angegebenen Bedeutungen haben, $R_1$ Wasserstoff oder $C_2$-$C_8$-Alkanoyl, insbesondere verzweigtes $C_3$-$C_6$-Alkanoyl, bedeutet, $R_2$ Acetyl, ist, X für $>N-$ steht, alk $C_1$-$C_7$-Alkylen, insbesondere $C_1$-$C_4$-Alkylen, bedeutet, $R_5$ Wasserstoff, $C_3$-$C_7$-Cycloalkyl, gegebenenfalls ein- oder mehrfach durch $C_1$-$C_4$-Alkyl oder Halogen substituiertes Phenyl, Biphenylyl oder Naphthyl bedeutet und $R_7$ Wasserstoff ist.

Die Erfindung betrifft insbesondere Verbindungen der Formel I und ihre Salze, worin die Strukturelemente $-A_1-A_2-$, $-A_3-A_4-$ und $-A_5-A_6-$ die angegebenen Bedeutungen haben, $R_1$ verzweigtes $C_3$-$C_6$-Alkanoyl, wie Pivaloyl, bedeutet, $R_2$ Acetyl ist, $R_3$ und $R_4$ die angegebenen Bedeutungen haben, X für $>N-$ steht, alk $C_1$-$C_4$-Alkylen, wie Methylen, bedeutet, $R_5$ $C_3$-$C_7$-Cycloalkyl, wie Cyclohexyl, $C_3$-$C_7$-Cycloalkenyl, wie Cyclohex-3-en-1-yl, oder durch $C_1$-$C_4$-Alkyl, wie Methyl, ein- oder mehrfach, wie zwei-oder dreifach, substituiertes Phenyl, wie 2-Methylphenyl, 2,3-, 2,5- oder 2,6-Dimethylphenyl oder 2,4,6-Trimethylphenyl, bedeutet und $R_7$ einerseits Wasserstoff, andererseits $C_1$-$C_4$-Alkyl, wie Methyl, bedeutet.

Die Erfindung betrifft insbesondere Verbindungen der Formel I und ihre Salze, worin die Strukturelemente $-A_1-A_2-$, $-A_3-A_4-$ und $-A_5-A_6-$, die Variablen $R_3$ und $R_4$ die angegebenen Bedeutungen haben, alk $C_1$-$C_4$-Alkylen, wie Methylen, 2,3-Propylen oder 2-Methyl-1,3-propylen, bedeutet, $R_1$ Wasserstoff oder verzweigtes $C_3$-$C_6$-Alkanoyl, wie Piovaloyl bedeutet, $R_2$ Acetyl ist, X für $>N-$ steht, $R_5$ Wasserstoff, $C_3$-$C_6$-Cycloalkyl, wie Cyclohexyl, Phenyl oder 2,4,6-Tri-$C_1$-$C_4$-alkyl-phenyl, wie 2,4,6-Trimethylphenyl, bedeutet und $R_7$ Wasserstoff ist.

Die Erfindung betrifft insbesondere Verbindungen der Formel I und ihre Salze, worin die Strukturelemente $-A_1-A_2-$, $-A_3-A_4-$ und $-A_5-A_6-$ die angegebenen Bedeutungen haben, X für $>N-$ steht, $R_2$ Wasserstoff oder Acetyle bedeutet, $R_3$ und $R_4$ gemeinsam für eine Bindung stehen, und einerseits alk $C_1$-$C_4$-Alkylen, in erster Linie 2-Methyl-1,3-propylen, bedeutet und $R_5$ Wasserstoff ist oder andererseits alk Methylen ist und $R_5$ 2,4,6,-Tri-$C_1$-$C_4$-alkyl-phenyl, in erster Linie 2,4,6-Trimethylphenyl, bedeutet und $R_7$ jeweils Wasserstoff ist.

Die Erfindung betrifft insbesondere Verbindungen der Formel I und ihre Salze, worin die Strukturelemente $-A_1-A_2-$, $-A_3-A_4-$ und $-A_5-A_6-$ die angegebenen Bedeutungen haben, X für $>N-$ steht, $R_2$ Acetyl bedeutet, $R_3$ und $R_4$ gemeinsam für eine Bindung oder jeweils für Wasserstoff stehen, und einerseits alk $C_1$-$C_4$-Alkylen, in erster Linie 2-methyl-1,3-propylen, bedeutet und $R_5$ Wasserstoff ist oder andererseits alk Methylen ist und $R_5$ 2,4,6-Tri-$C_1$-$C_4$-alkyl-phenyl, in erster Linie 2,4,6-Trimethylphenyl, bedeutet und $R_7$ jeweils $C_1$-$C_4$-Alkyl, wie Methyl, ist.

Die Erfindung betrifft in erster Linie Verbindungen der Formel I und ihre Salze, worin die Strukturelemente $-A_1-A_2-$, $-A_3-A_4-$ und $-A_5-A_6-$ jeweils Vinylen oder die Elemente $-A_1-A_2-A_3-A_4-$ Buta-1,3-dien-1,4-diyl und $-A_5-A_6-$Ethylen bedeuten, X für $>CH-$ steht, alk $C_1$-$C_4$-Alkylen, insbesondere Methylen oder 2,3-Propylen, bedeutet, $R_1$ verzweigtes $C_3$-$C_6$-Alkanoyl, insbesondere Pivaloyl, bedeutet, $R_2$ Acetyl ist, $R_3$ und $R_4$ gemeinsam für eine Bindung stehen und $R_5$ Wasserstoff, ferner Cyclohexyl oder Phenyl bedeutet und $R_7$ Wasserstoff ist.

Die Erfindung betrifft in erster Linie Verbindungen der Formel I und ihre Salze, worin die Strukturelemente $-A_1-A_2-$, $-A_3-A_4-$ und $-A_5-A_6-$ jeweils Vinylen oder die Elemente $-A_1-A_2-A_3-A_4-$ Buta-1,3-dien-1,4-diyl und $-A_5-A_6-$ Ethylen bedeuten, X für $>N-$ steht, $R_1$ Wasserstoff oder verzweigtes $C_3$-$C_6$-Alkanoyl, insbesondere Pivaloyl, bedeutet, $R_2$ Acetyl ist, $R_3$ und $R_4$ gemeinsam für eine Bindung stehen, alk $C_1$-$C_4$-Alkylen, insbesondere 2-Methyl-1,3-propyl, bedeutet und $R_5$ Wasserstoff ist oder alk $C_1$-$C_4$-Alkylen, insbesondere Methylen, bedeutet und $R_5$ 2,4,6-Tri-$C_1$-$C_4$-alkylphenyl, insbesondere 2,4,6-Trimethylphenyl, bedeutet und $R_7$ Wasserstoff ist.

Die Erfindung betrifft in erster Linie Verbindungen der Formel I und ihre Salze, worin X für $>N-$ steht, $-A_1-A_2-$ und $-A_3-A_4-$ jeweils Ethylen und $A_5-A_6-$ Vinylen oder Ethylen bedeuten, $R_1$ verzweigtes $C_3$-$C_6$-Alkanoyl, insbesondere Pivaloyl, bedeutet, $R_2$ Acetyl ist, $R_3$ und $R_4$ gemeinsam für eine Bindung oder jeweils für Wasserstoff stehen, einerseits alk $C_1$-$C_4$-Alkylen, insbesondere Methylen, bedeutet und $R_5$ 2,4,6-Trimethylphenyl bedeutet oder andererseits alk $C_1$-$C_4$-Alkylen, insbesondere 2-Methyl-1,3-propylen,

bedeutet und $R_5$ Wasserstoff bedeutet und $R_7$ Wasserstoff ist.

Die Erfindung betrifft in erster Linie Verbindungen der Formel I und ihre Salze, worin $-A_1-A_2-$, $-A_3-A_4-$ und $-A_5-A_6-$ jeweils Ethylen bedeuten, X für >N- steht, $R_1$ Pivaloyl bedeutet, $R_2$ Acetyl ist, $R_3$ und $R_4$ gemeinsam für eine Bindung oder jeweils Wasserstoff bedeuten, alk-$R_5$ 2,4,6-Trimethylbenzyl bedeutet und $R_7$ Wasserstoff ist.

Für die vorstehend definierten Verbindungen der Formel I und ihre Salze gelten ebenfalls die eingangs aufgeführten Ausschlussbedingungen.

Die Erfindung betrifft insbesondere die in den Beispielen genannten neuen Verbindungen und ihre Herstellung.

Ebenfalls Gegenstand der Erfindung sind Verfahren zur Herstellung der erfindungsgemässen Verbindungen. Die Herstellung vor Verbindungen der Formel I und ihrer Salze erfolgt in an sich bekannter Weise und ist z.B. dadurch gekennzeichnet, dass man,

a) zur Herstellung von Verbindungen der Formel I und ihrer Salze, worin X für >N-steht, eine Verbindung der Formel

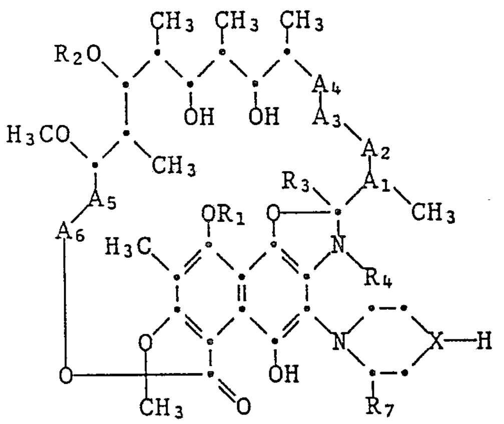

(IIa)

oder ein Salz davon, worin X für >N- steht, mit einer Verbindung der Formel

Z−alk−$R_5$ (IIb)

umsetzt, worin Z reaktionsfähiges verestertes Hydroxy bedeutet, oder,

b) zur Herstellung von Verbindungen der Formel I und ihrer Salze, worin $R_2$ Acetyl ist und $R_3$ und $R_4$ gemeinsam für eine Bindung stehen, eine Verbindung der Formel

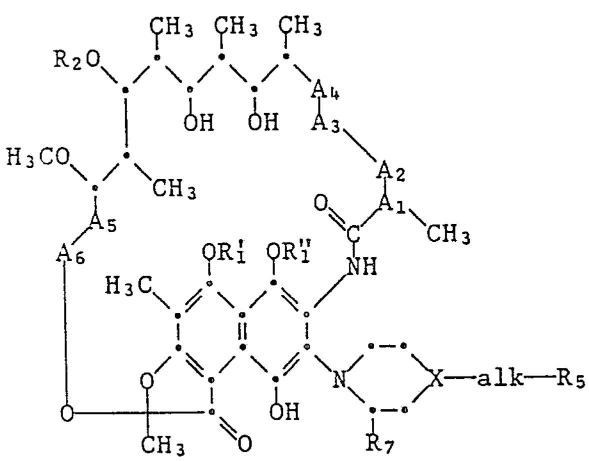

(III) ,

worin $R_1'$ Acyl bedeutet und $R_1''$ Wasserstoff oder Acyl bedeutet, cyclisiert, oder

c) eine Verbindung der Formel

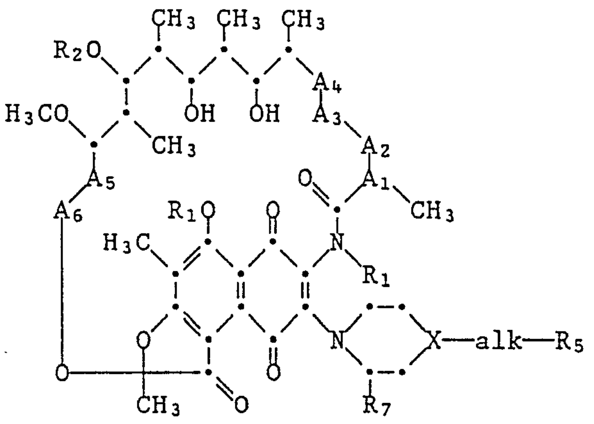

(IV)

cyclisiert,

und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt und, wenn erwünscht, ein verfahrensgemäss erhältliches Gemisch von Isomeren auftrennt.

Salze der Ausgangsmaterialien der Formeln IIa und III, die eine acide phenolische Hydroxygruppe aufweisen, sind entsprechende Salze mit Basen der vorstehend aufgeführten Art, während Ausgangsverbindungen der Formel IIb, die ein oder zwei basische Zentren aufweisen, entsprechende Säureadditionssalze analog der Säureadditionssalze der Formel I bilden können.

Reaktionsfähiges verestertes Hydroxy, z.B. Z, ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes $C_1$-$C_7$-Alkansulfonyloxy, z.B. Methan- oder Trifluormethansulfonyloxy, $C_5$-$C_7$-Cycloalkansulfonyloxy, z.B. Cyclohexansulfonyloxy, oder gegebenenfalls, z.B. durch $C_1$-$C_7$-Alkyl oder Halogen, substituiertes Benzolsulfonyloxy, z.B. p-Brombenzol- oder p-Toluolsulfonyloxy.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80° bis zur Siedetemperatur des Reaktionsmediums, vorzugsweise von etwa -10° bis etwa +180° C, und, falls erforderlich, in einem geschlossenen Gefäss, unter Druck, in einer Inertgasatmosphäre und/oder unter wasserfreien Bedingungen arbeitet.

Variante a):

Z bedeutet vorzugsweise Halogen, wie Chlor, Brom oder Iod, ferner Sulfonyloxy, wie Methan- oder p-Toluolsulfonyloxy.

Die Umsetzung erfolgt in an sich bekannter Weise, vorteilhaft in Gegenwart einer Base.

Als Basen kommen vorzugsweise nicht nucleophile tertiäre Amine in Frage, beispielsweise Triniederalkylamine, basische Heterocyclen und carbocyclische Amine, wie Ethyl-diisopropylamin, Triethylamin, Pyridin, 1,5-Diaza-bicyclo[4.3.0]non-5-en (DBN) sowie 1,8-Diaza-bicyclo[5.4.0]undec-7-en (DBU).

Das Ausgangsmaterial der Formel IIa kann in an sich bekannter Weise hergestellt werden, beispielsweise durch Umsetzung von 3-Halogen-rifamycin-S mit in Position 4 geschütztem Piperazin (z.B. analog US 4 005 077), anschliessender Acylierung zu entsprechenden 1-0-Acyl- bzw. 1,8-Di-0-acyl-rifamycin-SV-Derivaten und Cyclisierung gemäss Variante b). Schliesslich erfolgt die Abspaltung der Schutzgruppe.

Variante b) :

$R_1$ bzw. $R_1'$ Acyl steht in erster Linie für Pivaloyl.

Die Cyclisierung von Verbindungen der Formel III erfolgt vorteilhaft unter Erwärmen, z.B. in einem Temperaturbereich von etwa 50° C bis zur Siedetemperatur des Reaktionssystems, z.B. bis etwa 180° C, wie in einem Temperaturintervall von etwa 100° C bis etwa 170° C.

Die Cyclisierung von Verbindungen der Formel III beispielsweise, worin die Strukturelemente $-A_1-A_2-$, $-A_3-A_4-$ und $-A_5-A_6-$ Ethylen bedeuten, kann allerdings auch bei Raumtemperatur durchgeführt werden.

Das Ausgangsmaterial der Formel III kann man beispeilsweise herstellen, indem man Rifamycin-S oder 3-Halogen-, insbesondere 3-Brom-rifamycin-S mit einem Amin der Formel

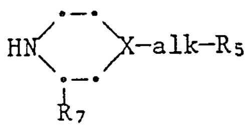

(IIIa)

umsetzt. Dabei arbeitet man insbesondere mit einem Ueberschuss an Amin der Formel IIIa beispielsweise in einem Temperaturbereich von etwa 0° bis etwa 100°C. Es bildet sich ein Gemisch aus der Chinon- und der Hydrochinonform. Dieses Gemisch kann mittels Reduktion, z.B. mit katalytischer Hydrierung, in das entsprechende Hydrochinon (Derivat von Rifamycin-SV) übergeführt werden ($R_1$ = H). Durch Behandeln mit entsprechenden Acylierungsmitteln, z.B. mit einem Säureanhydrid, wie Pivaloylchlorid, in Gegenwart einer Base, wie Pyridin, kann man zu Verbindungen der Formel III gelangen, worin $R_1'$ Acyl und $R_1''$ ≫ Wasserstoff bzw. $R_1$ und $R_1'$ Acyl bedeuten. Bedeuten die Strukturelemente $-A_1-A_2-$, $-A_3-A_4-$ und $-A_5-A_6-$jeweils Vinylen, können derartige Verbindungen (auch auf der Vorstufe) z.B. durch katalytische Hydrierung je nach Wahl der Reduktionsmittel und Aufnahme der $H_2$-Aequivalente in die entsprechende Tetrahydroform ($-A_1-A_2-$ und $-A_3-A_4-$ bedeuten jeweils Ethylen und $-A_5-A_6-$ ist Vinylen) oder Hexahydroform ($-A_1-A_2-$, $-A_3-A_4-$ und $-A_5-A_6-$ bedeuten jeweils Ethylen) übergeführt werden.

Variante c):

Die Cyclisierung erfolgt insbesondere durch Erwärmen oder Bestrahlung des Ausgangsmaterials.

Die Umsetzung wird vorzugsweise in einem organischen Lösungsmittel, beispielsweise einem Alkohol, wie Methanol, Aethanol oder Isopropanol, einem Keton, wie Aceton oder Methyläthylketon, einem chlorierten Kohlen wasserstoff, wie Chloroform oder Trichloräthan, einem Aether, wie Diäthyläther, einer Base, wie Pyridin oder Triäthylamin, oder einem Nitril, wie Acetonitril, durchgeführt. Bevorzugte Lösungsmittel sind Isopropanol oder Pyridin.

Falls die Reaktionstemperatur zu niedrig ist, verläuft die Umsetzung sehr langsam, wohingegen bei zu hoher Temperatur ein beträchtlicher Anteil an unerwünschten Nebenprodukten anfällt. Ein geeigneter Temperaturbereich liegt z.B. zwischen etwa 50° bis etwa 90°C, vorzugsweise bei etwa 75°C.

Die Bestrahlung wird in an sich bekannter Weise durchgeführt, beispielsweise unter Verwendung üblicher Bestrahlungsquellen, wie Mikrowellenbestrahlung.

Das resultierende Produkt kann gereinigt und isoliert werden, beispielsweise durch Chromatographie und/oder durch Umkristallisieren aus einem geeigneten Lösungsmittel, wie Petroläther.

Das Ausgangsmaterial der Formel IV kann in an sich bekannter Weise hergestellt werden, beispielsweise durch Behandeln einer Verbindung der Formel

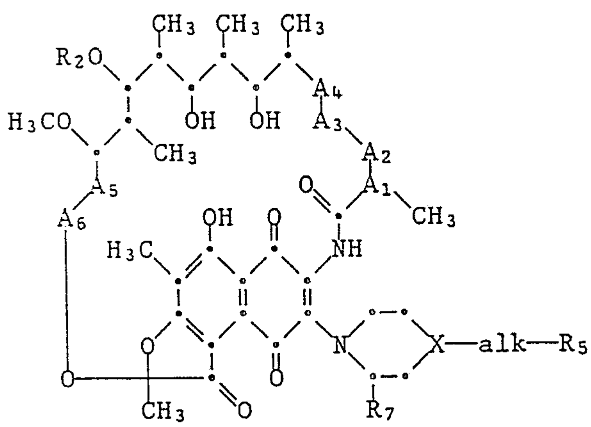

(IVa)

mit einem Acylierungsmittel, mit welchem die Triacetylgruppe in Position 8 und 14 eingeführt werden kann, wie Pivaloylchlorid.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I oder Salz davon kann in an sich bekannter Weise in eine andere Verbindung der Formel I übergeführt werden.

Verbindungen der Formel I, worin die Strukturelemente $-A_1-A_2-$, $-A_3-A_4-$und $-A_5-A_6-$ jeweils Vinylen oder $-A_1-A_2-$ und $-A_3-A_4-$ jeweils Ethylen und $-A_5-A_6-$ Vinylen bedeuten, können durch Reduktion, z.B. durch katalytische Hydrierung, in die entsprechenden Tetrahydro- ($-A_1-A_2-$ und $-A_3-A_4-$ sind jeweils Ethylen und $-A_5-A_6-$ ist Vinylen) oder die entsprechenden Hexahydroderivate ($-A_1-A_2-$, $-A_3-A_4-$ und $-A_5-A_6-$ bedeuten jeweils Ethylen) übergeführt werden. So erfolgt die Hydrierung der Mehrfachbindungen in Gegenwart von Hydrierungskatalysatoren, wobei hierfür z.B. Edelmetalle bzw. deren Derivate, z.B. Oxide, geeignet sind, wie Nickel, Raney-Nickel, Palladium, Platinoxid, die gegebenenfalls auf Trägermaterialien, z.B. auf Kohle oder Calciumcarbonat, aufgezogen sein können. Für die homogene Katalyse verwendet man insbesondere auch komplexe Rhodiumverbindungen als Katalysatoren, beispielsweise Tris-(triphenylphosphin)-rhodium(I)-chlorid. Die Hydrierung kann insbesondere bei Drucken von 1 bis etwa 100 at durchgeführt werden. Die entsprechenden Hexahydroderivate können durch katalytische Hydrierung weiter zu solchen Oktahydroderivaten der Formel I hydriert werden, worin $R_3$ und $R_4$ jeweils Wasserstoff bedeuten. Stehen $R_3$ und $R_4$ gemeinsam für eine Bindung, kann diese Doppelbindung auch in Gegenwart weiterer Doppelbindungen im Ansaring selektiv unter Verwendung eines geeigneten Hydrids, insbesondere Natriumborhydrid, hydriert werden. Die Reaktionsbedingungen können so gewählt werden, dass man zunächst 16,17,18,19-Tetrahydro-Derivate erhält (Katalysator z.B. Pd/C) und davon bzw. von entsprechend ungesättigten Ausgangsmaterialien ausgehend direkt zu den 16,17,18,19,28,29-Hexahydro-Verbindungen und dann durch Aufnahme eines weiteren $H_2$-Aequivalents zu den entsprechenden Oktahydro-Derivaten gelangt (Katalysator z.B. $PtO_2$). Unabhängig vom Hydrierungsgrad der Strukurelemente -$A_1$-$A_2$-, -$A_3$-$A_4$-, -$A_5$-$A_6$- können solche Verbindungen der Formel I, worin $R_3$ und $R_4$ gemeinsam für eine Bindung stehen, selektiv zu solchen Verbindungen der Formel I hydriert werden, worin $R_3$ und $R_4$ jeweils Wasserstoff bedeuten, beispielsweise durch Verwendung eines geeigneten Hydrids, insbesondere $NaBH_4$.

Bei der Hydrierungsreaktion können auch ungesättigte (cyclo-)aliphatische Kohlenwasserstoffelemente (alk bzw. $R_5$) gleichzeitig hydriert werden. Zur Vermeidung etwaiger unerwünschter Nebenreaktionen geht man vorteilhaft von entsprechenden hydrierten Verbindungen der Formel IIa aus und verfährt gemäss Variante a).

Steht alk bzw. der cycloaliphatische Kohlenwasserstoffrest $R_5$ in Verbindungen der Formel (I) für ungesättigte Kohlenwasserstoffreste, können die Mehrfachbindungen in an sich bekannter Weise gesättigt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet sind, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at durchgeführt werden.

Verbindungen der Formel I, worin $R_1$ Wasserstoff ist, können in an sich bekannter Weise acyliert werden, beispielsweise durch Umsetzung mit der entsprechenden Carbonsäure oder einem reaktionsfähigen Derivat davon. Derartige reaktionsfähige Derivate sind beispielsweise Anhydride, inklusive gemischte Anhydride, wie ein Säurehalogenid, z.B. -chlorid, oder Anhydride mit einem Ameisensäureester, aktivierte Carbonsäureester, wie Cyanmethyl-, (4-)Nitrophenyl-, Polyhalogenphenyl-, z.B. Pentachlorphenyl-, -ester. Die Umsetzung mit der Carbonsäure oder einem Salz davon erfolgt zweckmässig unter wasserabspaltenden Bedingungen, z.B. unter azeotroper Entfernung des Reaktionswassers, oder durch Behandeln mit einem geeigneten Kondensationsmittel, z.B. N,N′-Dicyclohexyl-carbodiimid. Die Umsetzung mit einem reaktionsfähigen Säurederivat wird vorteilhaft in Gegenwart einer Base durchgeführt. Entsprechend kann durch Behandeln mit einem entsprechenden Acetylierungsmittel der Acetylrest $R_2$ in Verbindungen der Formel I, worin $R_2$ Wasserstoff ist, eingeführt werden.

Durch Behandeln mit starken Basen, wie Alkalimetallhydroxiden, können der Acetylrest $R_2$ und der Acylrest $R_1$ durch Wasserstoff ersetzt werden. Der Acylrest $R_1$ kann auch in Gegenwart des Acetylrests $R_2$ selektiv abgespalten werden, beispielsweise durch Behandeln mit einem Fluorid, wie Alkalimetall-, z.B. Natrium- oder Cäsiumfluorid, oder einem Ammoniumfluorid, z.B. Tetrabutylammoniumfluorid.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung in freier Form und in Form ihrer Salze sind im Vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optischen Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines basischen Endstoffracemats mit einer optisch aktiven Säuren, wie Carbonsäure, z.B. Wein- oder Aepfelsäure, oder Sulfonsäure, z.B. Camphersulfonsäure, und Trennung des auf diese Weise erhaltenen

Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Bei der Hydrierung der $C_{16}$-$C_{17}$- und $C_{18}$-$C_{19}$-Doppelbindungen (-$A_1$-$A_2$- und -$A_3$-$A_4$- = Ethylen) entsteht ein zusätzliches asymmetrisch substituiertes C-Atom (C-16). Dementsprechend existieren hierzu zwei voneinander unterschiedliche stereoisomere Anordnungen am Atom C-16, die R- bzw. S-Konfiguration nach dem Cahn-Ingold-Prelog Nomenklatur System. Die Hydrierung der 1-Desoxy-15-desoxo-1,15-oxy-rifamycine verläuft im Hinblick auf C-16 praktisch stereospezifisch. Um zu bezüglich C-16 zu der anderen stereoisomeren Form zu gelangen, muss man beispielsweise von entsprechenden Hydroderivaten der Formel III ausgehen, worin $R_1'$ und $R_1''$ Wasserstoff bedeuten, die betreffenden stereoisomeren Formen auftrennen, beispielsweise durch übliche chromatographische Methoden, und anschliessend die so erhältlichen Verbindungen der Formel III entsprechend acylieren und die Cyclisierung z.B. nach Variante b) durchführen.

Aufgrund von Untersuchungen in der eingangs beschriebenen Testanordnung zur Bestimmung der lipidsenkenden Eigenschaften der erfindungsgemässen Verbindungen wurde festgestellt, dass eine der beiden bezüglich C-16 möglichen stereoisomeren Anordnungen für eine besonders hohe Hemmung des Cholesterinspiegels verantwortlich ist. Die absolute Konfiguration an C-16 für diese Stereoisomeren konnte bislang noch nicht ermittelt werden.

Dementsprechend betrifft die vorliegende Erfindung insbesondere solche der eingangs und in den Beispielen beschriebenen Verbindungen der Formel I und ihre Salze, in denen eine derartige stereoisomere Anordnung an C-16 vorliegt.

Die Erfindung betrifft auch diejenigen Ausführungsformen des Verfahrens, nach denen man von einer auf irgendeiner Stufe des Verfahrens also Zwischenprodukt erhältlichen Verbindung ausgeht und die fehlenden Schritte durchführt oder einen Ausgangsstoff in Form eines Derivates bzw. Salzes und/oder seiner Diastereomeren, Racemate bzw. Antipoden verwendet oder insbesondere unter den Reaktionsbedingungen bildet.

Die Aufarbeitung des Reaktionsproduktes und Isolierung aus dem verfahrensgemäss erhältlichen Reaktionsgemisch erfolgt in an sich bekannter Weise, z.B. durch Verdünnen mit Wasser, und/oder gegebenenfalls durch Neutralisieren oder leichtes Ansäuern (bis etwa pH = 3) mit einer wässrigen Säure, wie einer anorganischen oder organischen Säure, z.B. einer Mineralsäure oder, vorteilhafterweise, Zitronensäure, und Zugabe eines mit Wasser nicht-mischbaren Lösungsmittels, wie eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes der andere übliche Reinigungsmethoden in gereinigter Form erhalten werden kann. Ergibt die obige Reaktion beispielsweise ein Gemisch von acylierten Verbindungen bzw. diastereomeren Formen, kann dieses in an sich bekannter Weise, z.B. mittels fraktionierter Kristallisation, Chromatographie, etc. in die gewünschten individuellen Acyl-Verbindungen bzw. diastereomeren Formen aufgetrennt werden.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, insbesondere neue Verbindungen der Formel III, ihre Verwendung und Verfahren zu ihrer Herstellung bilden ebenfalls einen Gegenstand der Erfindung, wobei die Variablen $A_1$-$A_2$, $A_3$-$A_4$, $A_5$-$A_6$, $R_1$, $R_2$, $R_3$, $R_4$, $R_5$, $R_6$, $R_7$, X, und alk die für die jeweils bevorzugten Verbindungsgruppen der Formel I davon angegebenen Bedeutungen haben.

Die vorliegende Erfindung umfasst auch die Verwendung der Verbindungen der Formel I und ihrer Salze allein oder zusammen mit Hilfsstoffen, sowie auch in Kombination mit anderen Wirkstoffen, als Mittel zur therapeutischen, und zwar sowohl kurativen wie auch präventiven Behandlung von Krankheiten oder krankhaften Zuständen, die z.B. durch einen erhöhten Gehalt an Cholesterin und/oder Triglyceriden im Blut, insbesondere im Blutserum, angezeigt oder verursacht werden. Die erfindungsgemässen Wirkstoffe werden in therapeutisch wirksamen Mengen, vorzugsweise in Form von pharmazeutischen Zusammensetzungen zusammen mit konventionellen pharmazeutischen Trägermaterialien und/oder Hilfstoffen an den behandlungsbedürftigen Warmblüter, in erster Linie Menschen, verabreicht. Dabei werden z.B. an Warmblüter je nach Spezies, Körpergewicht, Alter und individuellem Zustand, tägliche Dosen entsprechend etwa 1 bis etwa 100, insbesondere etwa 3 bis etwa 50 mg pro kg Körpergewicht, die in schweren Fällen überschritten werden können, verabreicht. Sinngemäss umfasst die Erfindung auch die entsprechende Methode zur medizinischen Behandlung.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab.

Die neuen pharmazeutischen Präparate enthalten z.B. von etwa 10 % bis etwa 80 %, vorzugsweise von

etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetzes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol, Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulose-phthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Also Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche Viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Applikation eine ungefähre Tagesdosis von etwa 150 mg bis etwa 1500 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben. Die NMR-Spektren wurden zweckmässig bei erhöhten Temperaturen, insbesondere bei etwa 80° C und in DMSO aufgenommen; die chemischen Verschiebungen der Signale sind in ppm angegeben.

Die chemische Bezeichnung des Grundgerüsts, von dem sich die Rifamycin-Derivate der vorliegenden Erfindung ableiten, lautet wie folgt: 1-Desoxy-15-desoxo-1,15-oxy-rifamycin, und die zugehörige Strukturformel lässt sich folgendermassen darstellen:

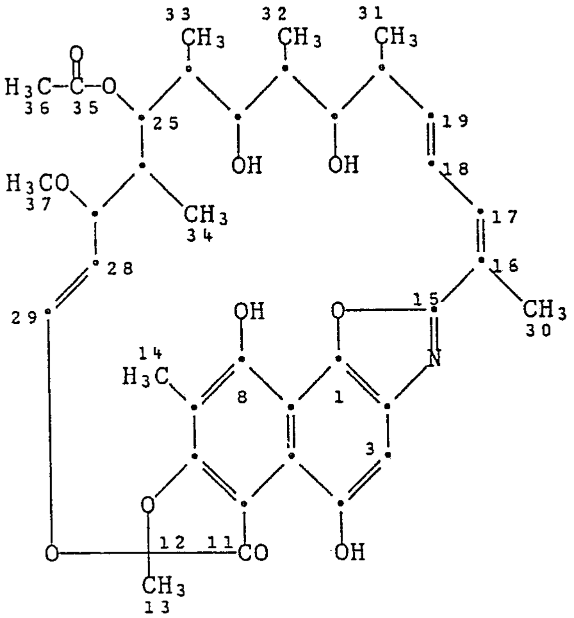

Beispiel 1:

Eine Lösung von 2 g 8-0-Pivaloyl-1-desoxy-15-desoxo-1,15-oxy-3-[4-(2,4,6-trimethylbenzyl)-piperazin-1-yl]-rifamycin der Formel I, worin die Strukturelemente $-A_1-A_2-$, $-A_3-A_4-$ und $-A_5-A_6-$ jeweils Vinylen bedeuten, X für $>N-$ steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist, hydriert man in 100 ml Aethanol in Gegenwart von 0,2 g Palladium auf Kohle (10 %) bei 25° und Normaldruck bis zum Ende der Wasserstoffaufnahme. Anschliessend entfernt man den Katalysator durch Filtration und dampft das Lösungsmittel im Vakuum ein. Der dunkelrote Rückstand wird an 200 g Kieselgel mit dem Elutionsmittel Petroläther/Essigester (3:1) chromatographiert. Man beobachtet zwei rasch wandernde rot gefärbte Banden, denen eine starke rote Hauptbande folgt. Das Eluat dieser roten Hauptbande wird gesammelt und eingedampft. Der Rückstand besteht aus epimerenreinem 16,17,18,19-Tetrahydro-8-O-pivaloyl-1-desoxy-15-desoxo-1,15-oxy-3-[4-(2,4,6-trimethybenzyl)-piperazin-1-yl)-rifamycin der Formel I, worin die Strukturelemente $-A_1-A_2-$ und $-A_3-A_4-$ jeweils Ethylen und $-A_5-A_6$ Vinylen bedeuten, X für $>N-$ steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist.

$C_{56}H_{77}N_3O_{12}$; MG: 983 (gefunden, MS). $^1$H-NMR (360 MHz, DMSO): Signal für $CH_3$-30 bei 1,4 ppm (d, 3H).

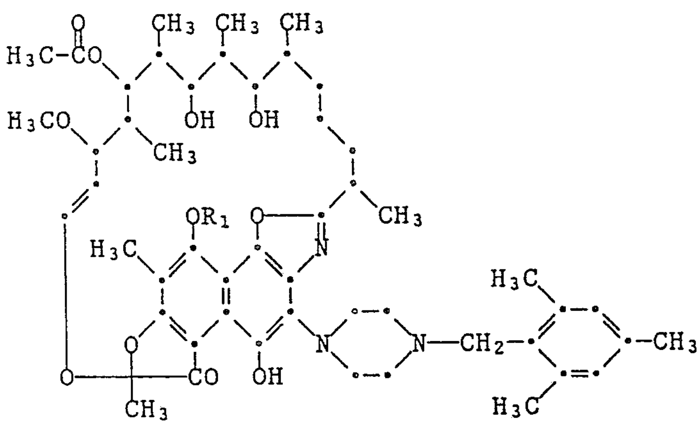

Das Ausgangsmaterial kann wie folgt hergestellt werden: Ein Gemisch aus 5 g 3-[4-(2,4,6-Trimethylbenzyl)-piperazin-1-yl]-rifamycin-SV [vgl. EP 244 398, Beispiel 2; die Herstellung weiterer, in den nachfolgenden Beispielen verwendeten Rifamycin-SV-Verbindungen wird beispielsweise in US 4 005 077 beschrieben], 50 ml trockenem Pyridin und 4,5 ml Pivaloylchlorid wird während 30 Minuten auf 50° gehalten. Anschliessend verdampft man das Lösungsmittel im Vakuum. Der ölige Rückstand wird in Essigester gelöst und mit 2N-Salzsäure, mit Pufferlösung pH = 7 und mit Kochsalzlösung gewaschen. Nach dem Trocknen über Natriumsulfat und Eindampfen wird der gelbe Rückstand aus Aether/Hexan kristallisiert. Man erhält so das

1,8-Di-O-pivaloyl-3-[4-(2,4,6-trimethylbenzyl)-piperazin-1-yl]-rifamycin-SV vom Schmelzpunkt 203-204°. $C_{61}H_{83}N_3O_{14}$; MG: 1081 (gefunden, MS).

In analoger Weise können die in den nachfolgenden Beispielen verwendeten 1,8-Di-0-pivaloylverbindungen hergestellt werden.

30 g 1,8-Di-O-pivaloyl-3-[4-(2,4,6-trimethylbenzyl)-piperazin-1-yl]-rifamycin-SV werden in der Wärme in 1000 ml 2-Methoxyäthanol gelöst und unter Stickstoff während 5 Stunden unter Rückfluss gekocht. Dann dampft man das Lösungsmittel im Vakuum ein und kristallisiert den Rückstand zweimal aus Methanol. Man erhält das 8-0-Pivaloyl-1-desoxy-15-desoxo-1,15-oxy-3-[4-(2,4,6-trimethylbenzyl)-piperazin-1-yl]-rifamycin der Formel I, worin die Strukturelemente -$A_1$-$A_2$-, -$A_3$-$A_4$- und -$A_5$-$A_6$-jeweils Vinylen bedeuten, X für >N- steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist. Schmelzpunkt 160-165° (Kristallisation aus Aethanol). $C_{56}H_{73}N_3O_{12}$; M = 979, gefunden (MS): 979; $^1$H-NMR (360 MHz, $CDCl_3$, TMS): 1,49 (s, 9H, Pivaloyl an O-8).

Beispiel 2:

Eine Lösung von 10 g des Zielprodukts von Beispiel 1 in 800 ml Aethanol wird in Gegenwart von 1,2 g Schwefelsäure und 1 g $PtO_2$ bei 25°C und Normaldruck während 1 Stunde hydriert. Nach dieser Zeit ist 1 Aequivalent Wasserstoff (230 ml) aufgenommen. Man entfernt den Katalysator durch Filtration, neutralisiert das Filtrat durch Zugeben von wässriger Natriumbicarbonatlösung, setzt gesättigte Kochsalzlösung zu und extrahiert das Hydrierungsprodukt durch wiederholtes Ausschütteln mit Essigester. Nach dem Trocknen und Eindampfen des Essigesterextraktes wird der Rückstand wie in Beispiel 1 beschrieben durch Chromatographie gereinigt. Das in der starken Hauptbande erhaltene rote Material stellt das epimerenreine 16,17,18,19,28,29-Hexahydro-8-O-pivaloyl-1-desoxy-15-desoxo-1,15-oxy-3-[4-(2,4,6-trimethylbenzyl)-piperazin-1-yl]-rifamycin der Formel I dar, worin die Strukturelemente -$A_1$-$A_2$-, -$A_3$-$A_4$- und -$A_5$-$A_6$-jeweils Ethylen bedeuten, X für >N- steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist. $C_{56}H_{79}N_3O_{12}$; MG: 985 (gefunden, MS,FD). $^1$H-NMR (360 MHz, DMSO): alle Signale vinylischer Protonen verschwunden.

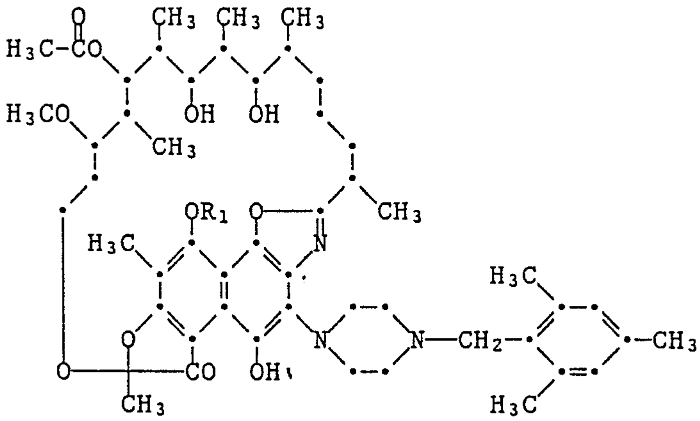

Beispiel 3:

In analoger Weise, z.B. wie in Beispiel 2 beschrieben, werden 5 g 8-0-Pivaloyl-1-desoxy-15-desoxo-1,15-oxy-3-[4-(2,4,6-trimethylbenzyl)-piperazin-1-yl]-rifamycin der Formel I, worin die Strukturelemente -$A_1$-$A_2$-, -$A_3$-$A_4$- und -$A_5$-$A_6$- jeweils Vinylen bedeuten, X für >N- steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist, in 500 ml Aethanol gelöst und in Gegenwart von 0,5 g $PtO_2$ und 0,7 g Schwefelsäure bei Raumtemperatur und Normaldruck bis zur Aufnahme von 4 Aequivalenten Wasserstoff (~ 460 ml) hydriert. Man entfernt den Katalysator durch Filtration, neutralisiert das Filtrat mittels wässriger Natriumbicarbonatlösung, setzt gesättigte Kochsalzlösung zu und extrahiert das Hydrierungsprodukt durch wiederholtes Ausschütteln mit Essigester. Nach dem Trocknen und Eindampfen des Essigesterextrakts wird der Rückstand in Aether gelöst. Nach einigem Stehen kristallisiert das N,15,16,17,18,19,28,29-Oktahydro-8-0-pivaloyl-1-desoxy-15-desoxo-1,15-oxy-3-[4-(2,4,6-trimethylbenzyl)-piperazin-1-yl]-rifamycin der Formel I, worin die Strukturelemente -$A_1$-$A_2$-, -$A_3$-$A_4$-und -$A_5$-$A_6$- jeweils Ethylen bedeuten, X für >N- steht, alk Methylen bedeutet, $R_1$ Pivaloyl bedeutet, $R_2$ Acetyl bedeutet, $R_3$ und $R_4$ jeweils Wasserstoff bedeuten, $R_5$ 2,4,6-Trimethylphenyl bedeutet und $R_7$ Wasserstoff ist. Die schwach braunroten Kristalle, die nach Umkristallisieren aus Aether farblose Plättchen bilden, schmelzen bei 226-227° (Zersetzung). $C_{56}H_{81}N_3O_{12}$; MG: 987 (gefunden; MS,FD).

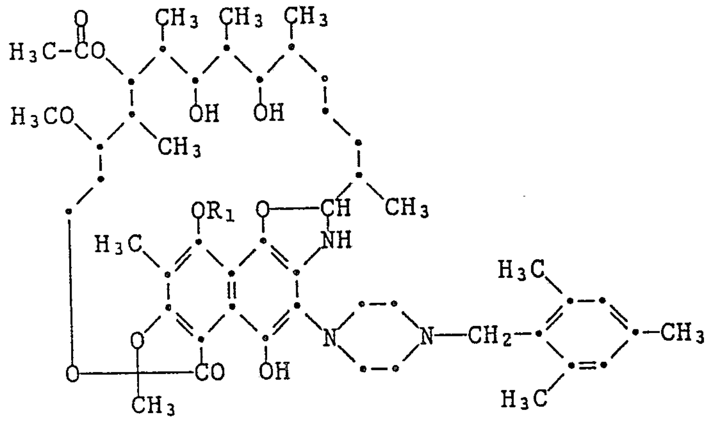

Beispiel 4

In analoger Weise, z.B. wie in Beispiel 1 bei der Herstellung des Ausgangsmaterials beschrieben, erhält man aus 1,8-Di-O-pivaloyl-3-[4-(1-naphthyl-methyl)-piperazin-1-yl]-rifamycin SV durch 5-stündiges Erhitzen in 2-Methoxyäthanol das 8-O-Pivaloyl-1-desoxy-15-desoxo-1,15-oxy-3-[4-(1-naphthyl-methyl)-piperazin-1-yl]-rifamycin der Formel I, worin die Strukturelemente $-A_1-A_2-$, $-A_3-A_4-$ und $-A_5-A_6-$ jeweils Vinylen bedeuten, X für $>$N- steht, alk Methylen bedeutet, $R_1$ Pivaloyl bedeutet, $R_2$ Acetyl bedeutet, $R_3$ und $R_4$ gemeinsam für eine Bindung stehen, $R_5$ 1-Naphthyl bedeutet und $R_7$ Wasserstoff ist, welches aus Methanol/Wasser in roten Kristallen kristallisiert. Schmelzpunkt 207°.

$C_{57}H_{69}N_3O_{12}$; MG: 987 (gefunden, MS,FD).

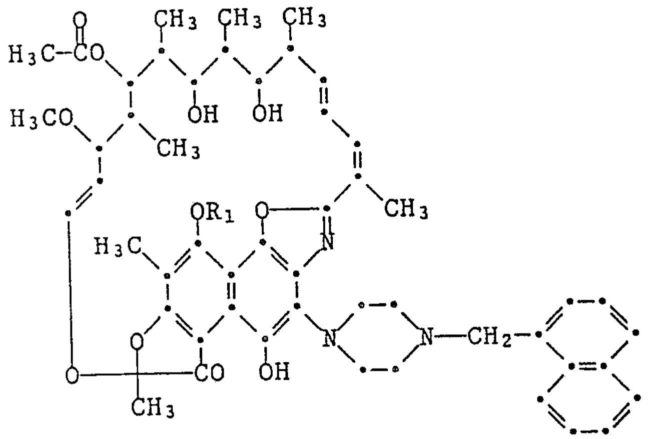

Beispiel 5:

In analoger Weise, z.B. wie in Beispiel 1 bei der Herstellung des Ausgangsmaterials beschrieben, erhält man aus 1,8-Di-O-pivaloyl-3-(4-isobutyl-1-piperazinyl)-rifamycin SV das 8-O-Pivaloyl-1-desoxy-15-desoxo-1,15-oxy-3-(4-isobutyl-1-piperazinyl)-rifamycin der Formel I, worin die Strukturelemente $-A_1-A_2-$, $-A_3-A_4-$ und $-A_5-A_6-$ jeweils Vinylen bedeuten, X für $>$N- steht, alk 2-Methyl-1,3-propylen bedeutet, $R_1$ Pivaloyl ist, $R_2$ Acetyl bedeutet, $R_3$ und $R_4$ gemeinsam für eine Bindung stehen, $R_5$ und $R_7$ Wasserstoff sind, vom Schmelzpunkt 187-188° (kristallisiert aus Methanol/Wasser).

$C_{50}H_{69}N_3O_{12}$; MG: 903 (gefunden, MS,FD). $^1H$-NMR (360 MHz, $CDCl_3$): Signale der Isobutylgruppe bei 0,92 (6H, $(CH_3)_2CH$); 2,16 (d, 2H, $CH_2N$).

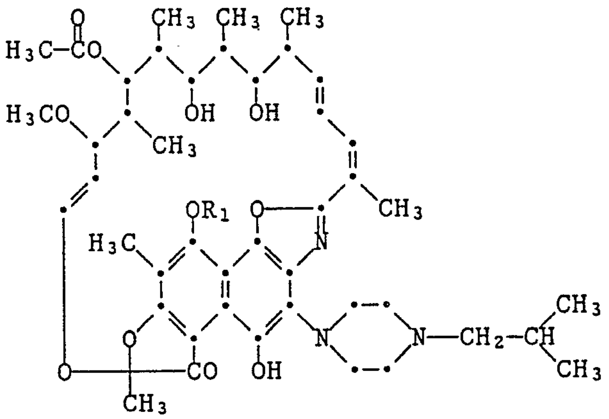

Beispiel 6:

In analoger Weise, z.B. wie in Beispiel 3 beschrieben, erhält man nach Hydrierung von 8-O-Pivaloyl-1-desoxy-15-desoxo-1,15-oxy-3-(4-isobutyl-1-piperazinyl)-rifamycin der Formel I, worin die Strukturelemente $-A_1-A_2-$, $-A_3-A_4-$ und $-A_5-A_6-$ jeweils Vinylen bedeuten, X für > N- steht, alk 2-Methyl-1,3-propylen bedeutet, $R_1$ Pivaloyl ist, $R_2$ Acetyl bedeutet, $R_3$ und $R_4$ gemeinsam für eine Bindung stehen, $R_5$ und $R_7$ Wasserstoff sind, mit $PtO_2$ in Ethanol unter Zusatz von Schwefelsäure das N,15,16,17,18, 19,28,29-Oktahydro-8-O-pivaloyl-1-desoxy-15-desoxo-1,15-oxy -3-(4-isobutyl-1-piperazinyl)-rifamycin der Formel I, worin die Strukturelemente $-A_1-A_2-$, $-A_3-A_4-$ und $A_5-A_6-$ jeweils Ethylen bedeuten, X für > N- steht, $R_1$ Pivaloyl ist, $R_2$ Acetyl bedeutet, $R_3$ und $R_4$ jeweils Wasserstoff bedeuten, $R_5$ und $R_7$ Wasserstoff sind und alk 2-Methyl-1,3-propylen bedeutet. Die schwach rötlich gefärbten Kristalle bilden nach Umkristallisieren aus Hexan farblose, quadratische Plättchen, die zwischen 150 und 160° schmelzen.

$C_{50}H_{77}N_3O_{12}$; MG; 911 (gefunden; MS, FD).

Beispiel 7:

In analoger Weise, z.B. wie in Beispiel 1 bei der Herstellung des Ausgangsmaterials beschrieben, erhält man ausgehend von 1,8-Di-O-pivaloyl-3-[4-(4-phenylbenzyl)-1-piperazinyl]-rifamycin-SV durch 5-stündiges Erhitzen in 2-Methoxyäthanol unter Stickstoff das 8-O-Pivaloyl-1-desoxy-15-desoxo-1,15-oxy-3-[4-(4-phenylbenzyl)-1-piperazinyl]-rifamycin der Formel I, worin die Strukturelemente $-A_1-A_2-$, $-A_3-A_4-$ und $A_5-A_6-$jeweils Vinylen bedeuten, X für > N- steht, alk Methylen bedeutet, $R_1$ Pivaloyl ist, $R_2$ Acetyl ist, $R_3$ und $R_4$ gemeinsam für eine Bindung stehen, $R_5$ 4-Biphenylyl bedeutet und $R_7$ Wasserstoff ist. Die Verbindung kristallisiert aus Hexan/Ether in roten Kristallen, die ab 140° schmelzen.

$C_{59}H_{71}N_3O_{12}$; MG: 1013 (gefunden: MS, FAB).

Beispiel 8:

Eine Lösung von 1 g 16,17,18,19-Tetrahydro-8-0O-pivaloyl-1-desoxy-15-desoxo-1,15-oxy-3-[4-(2,4,6-trimethylbenzyl)-1-piperazinyl]-rifamycin der Formel I, worin die Strukturelemente $-A_1-A_2-$, $-A_3-A_4-$jeweils Ethylen und $A_5-A_6-$ Vinylen bedeuten, X für > N- steht, alk Methylen bedeutet, $R_1$ Pivaloyl ist, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist, in 20 ml Methanol wird solange tropfenweise mit einer 5%igen methanolischen Lösung von $NaBH_4$ versetzt, bis die Reaktionslösung ihre rote Farbe verloren hat. Danach säuert man mit wässriger Ascorbinsäure an, setzt gesättigte NaCl-Lösung zu und nimmt das Reaktionsprodukt mit Essigsäureethylester auf. Nach dem Waschen des Essigesterextraktes mit Pufferlösung pH = 7, Trocknen über $Na_2SO_4$ und Eindampfen des Essigesterextraktes erhält man das N,15,16,17,18,19-Hexahydro-8-O-pivaloyl-1-desoxy-15-desoxo-1,15-oxy-3-(2,4,6-trimethylbenzyl)-1-piperazinyl]-rifamycin der Formel I, worin die Strukturelemente $-A_1-A_2-$und $A_3-A_4-$ jeweils Ethylen und $-A_5-A_6-$Vinylen bedeuten, alk Methylen bedeutet, $R_1$ Pivaloyl ist, $R_2$ Acetyl bedeutet, $R_3$ und $R_4$ und $R_7$ jeweils Wasserstoff bedeuten und $R_5$ 2,4,6-Trimethylphenyl bedeutet.

$C_{56}H_{79}N_3O_{12}$; MG: 985 (gefunden: MS, FD).

$^1H$-NMR (360 MHz, DMSO): 5,10 (dd, 1H, H-28) + 6,39 (d, 1H, H-29) [nur noch 2 Signale von vinylischen Protonen]; 5,79 (s, 1H, H-15); 1,39 (d über m, mindestens 3H, $CH_3$-30).

Beispiel 9:

Zu einer Lösung von 5 g 8-0-Pivaloyl-1-desoxy-15-desoxo-1,15-oxy-3-[4-(2,4,6-trimethylbenzyl)-1-piperazinyl]-rifamycin der Formel I, worin die Strukturelemente $-A_1-A_2-$, $-A_3-A_4-$ und $A_5-A_6-$ jeweils Vinylen bedeuten, X für > N- steht, alk Methylen bedeutet, $R_1$ Pivaloyl ist, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist, in 100 ml Tetrahydrofuran und 100 ml Methanol werden unter Rühren 0,2 g festes $NaBH_4$ gegeben. Nach 10 Minuten wird die blassgelbe Reaktionslösung mit wässriger Ascorbinsäurelösung angesäuert, mit Wasser und gesättigter, wässriger

NaCl-Lösung versetzt. Das Reaktionsgemisch wird mit Essigsäureethylester aufgenommen, der Extrakt mit Pufferlösung pH = 7 gewaschen, über $Na_2SO_4$ getrocknet und eingedampft. Der ölige Rückstand kristallisiert aus Aether/Pentan. Man erhält so das N,15-Dihydro-8-0-pivaloyl-1-desoxy-15-desoxo-1,15-oxy-3-[4-(2,4,6-trimethylbenzyl)-1-piperazinyl]-rifamycin der Formel I, worin die Strukturelemente $-A_1-A_2-$, $-A_3-A_4-$ und $-A_5-A_6-$ jeweils Vinylen bedeuten, X für > N- steht, alk Methylen bedeutet, $R_1$ Pivaloyl ist, $R_2$ Acetyl bedeutet, $R_3$ und $R_4$ und $R_7$ jeweils Wasserstoff bedeuten und $R_5$ 2,4,6-Trimethylphenyl bedeutet. Die Substanz bildet blassgelbliche Kristalle vom Smp. 205°.
$C_{56}H_{75}N_3O_{12}$; MG: 981 (gefunden, MS, FD).
$^1$H-NMR (360 MHz, $CDCl_3$, 50°): 5,78 (s, 1H, H-15).

Beispiel 10:
In analoger Weise, z.B. wie in Beispiel 1 bei der Herstellung des Ausgangsmaterials beschrieben, erhält man aus 1,8-Di-O-pivaloyl-3-[4-(2,5-dimethylbenzyl)-1-piperazinyl]-rifamycin das 8-O-Pivaloyl-1-desoxy-15-desoxo-1,15-oxy-3-[4-(2,5-dimethylbenzyl)-1-piperazinyl]-rifamycin der Formel I, worin die Strukturelemente $-A_1-A_2-$, $-A_3-A_4-$ und $-A_5-A_6-$ jeweils Vinylen bedeuten, X für > N- steht, alk Methylen bedeutet, $R_1$ Pivaloyl ist, $R_2$ Acetyl bedeutet, $R_3$ und $R_4$ gemeinsam für eine Bindung stehen, $R_5$ 2,5-Dimethylphenyl ist und $R_7$ Wasserstoff ist, vom Schmelzpunkt 220° (kristallisiert aus Methanol).
$C_{55}H_{71}N_3O_{12}$; MG: 965 (gefunden, MS, DCI). $^1$H-NMR. Spektrum (360 MHz, DMSO-$d_6$): Signale der 2,5-Dimethylbenzylgruppe bei 3,48 ($-CH_2-$), 2,27 und 2,32 (arom. $CH_3$) sowie 7,09 (1 arom. H) und 7,0 (zentr. AB, 2 arom. H) ppm.

Beispiel 11:
In analoger Weise, z.B. wie in Beispiel 1 bei der Herstellung des Ausgangsmaterials beschrieben, erhält man ausgehend von 1,8-Di-O-pivaloyl-3-(4-benzyl-1-piperazinyl)-rifamycin das 8-O-Pivaloyl-1-desoxy-15-desoxo-1,15-oxy-3-(4-benzyl-1-piperazinyl)-rifamycin der Formel I, worin die Strukturelemente $-A_1-A_2-$, $-A_3-A_4-$ und $-A_5-A_6-$ jeweils Vinylen bedeuten, X für > N- steht, $R_1$ Pivaloyl ist, $R_2$ Acetyl bedeutet, $R_3$ und $R_4$ gemeinsam für eine Bindung stehen, alk Methylen bedeutet, $R_5$ Phenyl ist und $R_7$ Wasserstoff ist. Die Verbindung bildet aus Methanol/Wasser dünne Kristallplättchen, die bei 181-182° schmelzen.
$C_{53}H_{67}N_3O_{12}$; MG: 937 (gefunden: MS, FAB).

Beispiel 12:
In analoger Weise, z.B. wie in Beispiel 1 bei der Herstellung des Ausgangsmaterials beschrieben, erhält man ausgehend von 1,8-Di-O-pivaloyl-3-(4-cyclohexylmethyl-1-piperazinyl)-rifamycin das 8-O-Pivaloyl-1-desoxy-15-desoxo-1,15-oxy-3-(4-cyclohexylmethyl-1-piperazinyl)-rifamycin der Formel I, worin die Strukturelemente $-A_1-A_2-$, $-A_3-A_4-$ und $-A_5-A_6-$ jeweils Vinylen bedeuten, X für > N- steht, alk Methylen bedeutet, $R_1$ Pivaloyl ist, $R_2$ Acetyl ist, $R_3$ und $R_4$ gemeinsam für eine Bindung stehen, $R_5$ Cyclohexyl ist und $R_7$ Wasserstoff ist. Die Verbindung kristallisiert aus Methanol in Plättchen, die bei 155-156° schmelzen.
$C_{53}H_{73}N_3O_{12}$; MG: 943 (gefunden: MS, DCI).

Beispiel 13:
In analoger Weise, z.B. wie in Beispiel 1 bei der Herstellung des Ausgangsmaterials beschrieben, erhält man ausgehend von 1,8-Di-0-pivaloyl-3-[4-(cyclohex-3-en-1-yl-methyl)-1-piperazinyl]-rifamycin-SV das 8-0-Pivaloyl-1-desoxy-15-desoxo-1,15-oxy-3-[4-(cyclohex-3-en-1-yl-methyl)-1-piperazinyl]-rifamycin der Formel I, worin die Strukturelemente $-A_1-A_2-$, $-A_3-A_4-$ und $-A_5-A_6-$ jeweils Vinylen bedeuten, X für > N- steht, alk Methylen bedeutet, $R_1$ Pivaloyl ist, $R_2$ Acetyl ist, $R_3$ und $R_4$ gemeinsam für eine Bindung stehen, $R_5$ Cyclohex-3-en-1-yl bedeutet und $R_7$ Wasserstoff ist. Die Verbindung kristallisiert aus Methanol in rhomboidischen Plättchen, die unter vorherigem Sintern bei ~165° schmelzen.
$C_{53}H_{71}N_3O_{12}$; MG: 941 (gefunden: MS, DCI).
$^1$H-NMR (360 MHz, DMSO-$d_6$): 5,66 (s, 2H, 2 olef. H in cyclohexen), 2,26 (t, $>N-CH_2-CH<$ überlagert mit s bei 2,24 von $CH_3$-30) ppm.

Das Ausgangsmaterial kann folgendermassen hergestellt werden:

a) Eine Lösung von 5 g 3-Bromrifamycin-S in 50 ml Tetrahydrofuran wird mit 2 g N-(Cyclohex-3-en-1-yl-methyl)-piperazin versetzt und bei 20° während 30 Minuten stehengelassen. Danach säuert man durch Zugabe von wässriger Zitronensäurelösung an und nimmt das Reaktionsprodukt in Essigester auf. Nach dem Trocknen und Eindampfen des Essigesterextraktes bleibt ein dunkler Rückstand. Man löst diesen in Methanol und setzt tropfenweise wässrige Ascorbinsäurelösung zu. Dabei färbt sich die Lösung gelb und nach kurzer Zeit fällt das 3-[4-(Cyclohex-3-en-1-yl-methyl)-piperazin-1-yl]-rifamycin-SV in gelbgefärbten Kristallen aus, die bei 170-172° schmelzen.

b) Ein Gemisch aus 5 g 3-[4-Cyclohex-3-en-1-yl-methyl)-1-piperazinyl]-rifamycin-SV, 50 ml trockenem Pyridin und 4,5 ml Pivaloylchlorid wird wäshrend 30 Minuten auf 50° erwärmt. Anschliessend verdampft man das Lösungsmittel im Vakuum. Der ölige Rückstand wird in Essigester gelöst und mit 2N-Salzsäure, mit Pufferlösung pH = 7 und mit Kochsalzlösung gewaschen. Nach dem Trocknen und Eindampfen wird ein gelber Rückstand von rohem 1,8-Di-0-pivaloyl-3-[4-(cyclohex-3-en-1-yl-methyl)-1-piperazinyl]-rifamycin-SV erhalten, der ohne weitere Reinigung weiterverarbeitet wird.

Beispiel 14:

Eine methanolische Lösung von 8-O-Pivaloyl-1-desoxy-15-desoxo-1,15-oxy-3-[4-(2,4,6-trimethylbenzyl)-1-piperazinyl]-N,15,16,17,18,19,28,29-oktahydrorifamycin der Formel I, worin die Strukturelemente $-A_1-A_2-$, $-A_3-A_4-$ und $-A_5-A_6-$ jeweils Aethylen bedeuten, X für >N- steht, alk Methylen bedeutet, $R_1$ Pivaloyl bedeutet, $R_2$ Acetyl bedeutet, $R_3$ und $R_4$ und $R_7$ jeweils für Wasserstoff stehen und $R_5$ 2,4,6-Trimethylphenyl bedeutet (Zielprodukt von Beispiel 3), wird mit überschüssiger wässriger Lösung von L(+)-Ascorbinsäure angesäuert, mit Wasser stark verdünnt und mit Essigester ausgeschüttelt. Dabei tritt das Ascorbinsäure-Salz des Oktahydroderivats in die Essigesterphase. Nach dem Trocknen über $Na_2SO_4$ und Eindampfen der Essigesterlösung bleibt als farbloser Lack das Ascorbat von 8-0-Pivaloyl-1-desoxy-15-desoxo-1,15-oxy--3-[4-(2,4,6-trimethylbenzyl)-1-piperazinyl]-N,15,16,17,18,19,28,29-oktahydrorifamycin der Formel I zurück in der die Strukturelemente $-A_1-A_2-$, $-A_3-A_4-$ und $-A_5-A_6-$ jeweils Aethylen bedeuten, X für >N- steht, alk Methylen bedeutet, $R_1$ Pivaloyl ist, $R_2$ Acetyl ist, $R_3$ und $R_4$ und $R_7$ jeweils für Wasserstoff stehen und $R_5$ 2,4,6-Trimethylphenyl ist. Die Verbindung kristallisiert aus Essigester bei Aetherzusatz in langen, dünnen Prismen, die bei etwa 200° unter Zersetzung schmelzen.

Beispiel 15:

Auf analoge Weise, z.B. wie in Beispiel 14 beschrieben, erhält man aus 8-0-Pivaloyl-1-desoxy-15-desoxo-1,15-oxy-3-[4-(2,4,6-trimethylbenzyl)-1-piperazinyl]-N,15,16,17,18,19-hexahydrorifamycin-SV (Zielprodukt von Beispiel 8) das Ascorbinsäure-Salz der Verbindung der Formel I, in der die Struktkurelemente $-A_1-A_2-$, $-A_3-A_4-$ jeweils Aethylen und $-A_5-A_6-$ Vinylen bedeutet, X für >N- steht, alk Methylen bedeutet, $R_1$ Pivaloyl ist, $R_2$ Acetyl ist, $R_3$ und $R_4$ und $R_7$ jeweils für Wasserstoff stehen und $R_5$ 2,4,6-Trimethylphenyl ist. Das Salz bildet farblose Prismen vom Smp. 175° (Zersetzung).

Beispiel 16:

In einer Lösung von 10 g 16,17,18,19,28,29-Hexahydro-3-[4-(2,4,6-trimethylbenzyl)-1-piperazinyl]-rifamycin (Diastereomerengemisch) in 100 ml Pyridin wird unter Rühren bei 0° 10 ml Pivaloylchlorid eingetropft und die Reaktionslösung 4 Stunden bei Raumtemperatur stehen gelassen. Anschliessend setzt man Wasser zu, säuert mit verdünnter Salzsäure an und nimmt das Reaktionsprodukt in Essigester auf. Der Essigesterextrakt wird mit verdünnter Säure, mit Pufferlösung pH = 7 und mit gesättigter Kochsalzlösung gewaschen, dann über $Na_2SO_4$ getrocknet und im Vakuum eingedampft. Der Rückstand enthält die 2 diastereomeren, rotgefärbten Hexahydroderivate, die durch Chromatographie getrennt werden: bei der wiederholten Säulenchromatographie an 1 kg Kieselgel mit dem Elutionsmittel n-Hexan/Essigester 3:1 isoliert man die in diesem System rascher wandernde von 2 rotgefärbten Banden. Sie enthält das diastereomerenreine 16,17,18,19,28,29-Hexahydro-8-0-pivaloyl-1-desoxy-15-desoxo-1,15-oxy-3-[4-(2,4,6-trimethylbenzyl)-piperazin-1-yl]-rifamycin der Formel I worin die Strukturelemente $-A_1-A_2-$, $-A_3-A_4-$ und $-A_5-A_6-$ jeweils Aethylen bedeuten, X für >N- steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist, welches diastereomer zu dem Zielprodukt von Beispiel 2 ist.

$C_{56}H_{79}N_3O_{12}$; MG: 985 (gefunden: MS, FAB).

Das Ausgangsmaterial kann beispielsweise wie folgt hergestellt werden: Eine Lösung von 3 g 3-[4-(2,4,6-Trimethylbenzyl)-piperazin-1-yl]-rifamycin-SV in 300 ml Aethanol wird mit 0,5 g $PtO_2$ versetzt, während 13 Stunden bei Raumtemperatur und unter Normaldruck hydriert. Anschliessend wird der Katalysator durch eine Schicht Kieselgur abfiltriert, das Filtrat zur Trockene eingedampft und der Rückstand in einem Gemisch aus Aethylacetat und Diäthyläther kristallisiert. Man erhält so das 3-[4-(2,4,6-Trimethylbenzyl)-piperazin-1-yl]-16,17,18,19,28,29-hexahydro-rifamycin-SV (Epimerengemisch) in Form von gelbgefärbten Kristallen, die sich oberhalb 250° zersetzen; Massenspektrum: m/z =920 ($M^+$ + I) entsprechend der Summenformel $C_{51}H_{73}N_3O_{12}$.

Beispiel 17:

Zu einer Lösung von 3 g des Zielproduktes von Beispiel 16 in 50 ml Methanol wird bei Raumtemperatur unter Rühren solange eine etwa 1%ige Lösung von $NaBH_4$ in Methanol langsam zugetropft, bis die anfangs tiefrote Farbe des Reaktionsgemisches völlig verschwunden ist und das Gemisch eine blassgelbe Farbe angenommen hat. Man verdünnt nun mit viel Wasser, nimmt das reduzierte Material in Aether auf, wäscht die Aether lösung mit Pufferlösung pH = 7 und mit gesättigter Kochsalzlösung, trocknet mit $Na_2SO_4$ und dampft ein. Der fast farblose Rückstand wird mehrmals aus Aether kristallisiert. Man erhält so das N,15,16,17,18,28,29-Oktahydro-8-0-pivaloyl-1-desoxy-15-desoxo-1,15-oxy-3-[4-(2,4,6-trimethylbenzyl)-piperazin-1-yl]-rifamycin der Formel I, worin die Strukturelemente $-A_1-A_2-$, $-A_3-A_4-$ und $-A_5-A_6-$ jeweils Aethylen bedeuten, X für >N- steht, alk Methylen bedeutet, $R_1$ Pivaloyl bedeutet, $R_2$ Acetyl bedeutet, $R_3$ und $R_4$ jeweils Wasserstoff bedeuten und $R_5$ 2,4,6-Trimethylphenyl bedeutet, welches diastereomer zu dem Zielprodukt von Beispiel 3 ist. Die farblosen Kristalle schmelzen bei 234-235°. $C_{56}H_{81}N_3O_{12}$; MG: 987 (gefunden: MS, DCI). $^1$H-NMR.-Spektrum (360 MHz, DMSO-$d_6$, ppm): keine vinylischen H-Signale mehr vorhanden; Unterschiede zum Spektrum der diastereomeren Verbindung (Zielprodukt von Beispiel 3) nur im Bereich hohen Feldes, z.B.: -0,28 (d, 3H); 0,29 (d, 3H); 0,60 (d, 3H) und 0,78 (d, 3H, 4 Ansaringmethylgruppen); 1,40 (d, ~3H, H-30).

Beispiel 18:

Eine 10%ige Lösung von frisch hergestelltem 8-0-Pivaloyl-3-[4-(2,4,6-trimethylbenzyl)-1-piperazinyl]-rifamycin-SV in Toluol wird im Druckgefäss während 15 Minuten auf 170° erhitzt. Dann wird das Toluol verdampft. Das zurückbleibende Material kristallisiert aus Methanol/Wasser. Die nach zweimaliger Kristallisation erhaltenen Kristalle vom Smp. 175° stellen das 1-Desoxy-15-desoxo-1,15-oxy-3-[4-(2,4,6-trimethylbenzyl)-1-piperazinyl]-rifamycin der Formel I dar, worin die Strukturelemente -$A_1$-$A_2$, -$A_3$-$A_4$-und -$A_5$-$A_6$- jeweils Vinylen bedeuten, X für $>$N- steht, $R_1$ Wasserstoff bedeutet, $R_2$ Acetyl bedeutet, $R_3$ und $R_4$ gemeinsam für eine Bindung stehen, alk Methylen bedeutet, $R_5$ 2,4,6-Trimethylphenyl bedeutet und $R_7$ Wasserstoff ist.

Das Ausgangsmaterial kann z.B. folgendermassen hergestellt werden:

a) Eine Lösung von 10 g 3-[4-(2,4,6-Trimethylbenzyl)-1-piperazinyl]-rifamycin-S in 100 ml Pyridin wird unter Rühren tropfenweise mit 1,5 g Pivalinsäurechlorid (1,13 Aequiv.) versetzt und bei 20° während 10 Minuten reagieren gelassen. Anschliessend setzt man dem Reaktionsgemisch 10 ml Methanol zu und rührt noch 1 Stunde weiter. Dann wird im Vakuum zur Trockne eingedampft, der Rückstand im Essigester gelöst, die Essigesterlösung mit wässriger Natriumbicarbonatlösung und gesättigter Kochsalzlösung gewaschen, über $Na_2SO_4$ getrocknet und eingedampft. Es bleibt 8-O-Pivaloyl-3-[4-(2,4,6-trimethylbenzyl)-1-piperazinyl]-rifamycin-S zurück, das aus Aether blauschwarze Kristalle vom Smp. 191-193° (Zersetzung) bildet. (Sintern bei etwa 162°, sowie wiederum bei etwa 175°.)

b) Das erhaltene Chinon wird in Tetrahydrofuran gelöst und der Lösung unter gutem Rühren ein Ueberschuss Zinkstaub und tropfenweise solange 1N Salzsäure zugesetzt, bis das Reaktionsgemisch eine gelbe Farbe angenommen hat. Nun filtriert man das Reaktionsgemisch, wäscht die Tetrahydrofuranlösung zweimal mit gesättigter Kochsalzlösung, trocknet sie mit Natriumsulfat und dampft im Vakuum rasch bei niedriger Temperatur ein. Der gelbe Rückstand besteht aus 8-0-Pivaloyl-3-[4-(2,4,6-trimethylbenzyl)-1-piperazinyl]-rifamycin-SV, welches in dieser Form direkt für die Ringschlussreaktion eingesetzt werden kann. Das Material kristallisiert aus Aether in orangegelben Kristallen, die bei etwa 165° unter Zersetzung schmelzen.

Beispiel 19:

Eine Lösung von 1,8-Di-O-pivaloyl-3-[2-methyl-4-(2,4,6-trimethylbenzyl)-piperazin-1-yl]-rifamycin in 8 ml 2-Methoxyäthanol wird unter Stickstoff unter Rückfluss erhitzt. Nach 5stündigem Erhitzen wird das Lösungsmittel im Vakuum entfernt. Das Rohprodukt wird durch flash-Chromatographie an Silikagel gereinigt, eluiert mit Ethylacetat/Hexan = 1:4 bis 2:3. Man erhält das 8-O-Pivaloyl-1-deoxy-15-desoxo-1,15-oxy-3-[2-methyl-4-(2,4,6-trimethylbenzyl)-piperazin-1-yl]-rifamycin der Formel I, worin die Strukturelemente -$A_1$-$A_2$-, -$A_3$-$A_4$- und -$A_5$-$A_6$-jeweils Vinylen bedeuten, $R_1$ Pivaloyl bedeutet, $R_2$ Acetyl bedeutet, $R_3$ und $R_4$ gemeinsam für eine Bindung stehen, alk Methylen bedeutet, $R_5$ 2,4,6-Trimethylphenyl bedeutet und $R_7$ Methyl ist.

Isomeres A

(im Hinblick auf das $R_7$ (= Methyl) aufweisende C-Atom des Piperazinringes): Schmelzpunkt: 140° (Zers.), NMR (300 MHz, $CD_3OD$): 6,80 (s, 2H).

Isomeres B

(im Hinblick auf das $R_7$ (= Methyl) aufweisende C-Atom des Piperazinringes): Schmelzpunkt: 140° (Zers.), NMR (300 MHz, $CD_3OD$): 6,85 (s, 2H).

Das Ausgangsmaterial kann wie folgt hergestellt werden:

Zu einer Lösung von 4,45 g (5,75 mMol) 3-Brom-rifamycin-S in 160 ml Tetrahydrofuran wird bei Raumtemperatur das Gemisch aus 1,34 g (5,75 mMol) 1-Trimethyl-3-methyl-piperazin und 1,17 ml (8,17 mMol) Triäthylamin in 160 ml Tetrahydrofuran bei schneller Tropfgeschwindigkeit zugetropft. Nach 10 Minuten zeigt die Dünnschichtchromatographie kein Ausgangsmaterial im Reaktionsgemisch mehr. Das Lösungsmittel wird unter vermindertem Druck entfernt, und es fällt ein dunkler, fester Rückstand an, der in einer Silikagel flash-Säule mit Methylenchlorid und 4 % Methanol als Eluiermittel chromatographiert wird. Man erhält so das 3-[2-Methyl-4-(2,4,6-trimethylbenzyl)-piperazin-1-yl]-rifamycin S.

NMR (270 MHz, $CD_3OD$): 6,78 (2H, s).

Eine Lösung von 2,14 g (109,8 mMol) Natriumascorbat in Methanol/Wasser (jeweils 10 ml) wird zu einer Lösung von 1 g (1,08 mMol) 3-[2-Methyl-4-(2,4,6-trimethylbenzyl)-piperazin-yl]-rifamycin-S in 40 ml Methanol bei Raumtemperatur zugetropft. Nachdem die Zugabe beendet ist, wird das Reaktionsgemisch 1 Stunde gerührt, wobei die Farbe von rot nach orange wechselt. Dünnschichtchromatographie in 10 % Methanol/Methylenchlorid zeigt kein Ausgangsmaterial mehr. Unlösliches wird abfiltriert, und das Filtrat wird fast zur Trockene eingedampft. Der Rückstand wird zwischen Methylenchlorid und gesättigter $NaHCO_3$-Lösung verteilt. Der organische Extrakt wird mit Salzlösung gewaschen, über $MgSO_4$ getrocknet und konzentriert. Man erhält so die Hydrochinon-Form (SV) als Diastereomerenpaar bezüglich des $R_7$ (=Methyl) aufweisenden C-Atoms des Piperazinringes als einen gelben Feststoff.

NMR (300 MHz, $CD_3OD$): 6,99 (s, 2H, Isomeres A); 6,95 (s, 2H, Isomeres B).

Zu einer Lösung von 1,02 g (1,08 mMol) 3-[2-Methyl-4-(2,4,6-trimethylbenzyl)-piperazin-1-yl]-rifamycin-SV in 20 ml Methylenchlorid wird bei 0° 25 mg Dimethylaminopyridin und 0,32 ml Triäthylamin zugegeben und anschliessend tropfenweise 0,27 ml (2,20 mMol) Pivaloylchlorid in 6 ml Methylenchlorid zugesetzt. Das

Reaktionsgemisch wird mit gesättigter $NaHCO_3$-Lösung, NaCl-Lösung gewaschen, über $MgSO_4$ getrocknet und konzentriert. Das Rohprodukt mittels flash-Chromatographie an Silikagel getrennt, wobei als Eluiermittel Ethylacetat/Hexan 3:1 verwendet wird. Man erhält so bezüglich des $R_7$ (= Methyl) aufweisenden C-Atoms des Piperazinringes 2 Isomere von 1,8-Di-O-pivaloyl-3-[2-methyl-4-(2,4,6-trimethylbenzyl)-piperazin-1-yl]-rifamycin.

Isomeres A:
NMR (270 MHz, $CD_3OD$): 6,8 (s, 2H); 1,81 (s, 3H);
$R_f$ = 0,76 Ethylacetat/Hexan 1:1)

Isomeres B:
NMR (270 MHz, $CD_3OD$): 6,75 (s, 2H); 1,7 (s, 3H);
$R_f$ = 0,71 Ethylacetat/Hexan 1:1).

Beispiel 20:

Eine Lösung von 50 mg des Isomeren A gemäss Beispiel 19, 10 mg $PtO_2$, 0,02 ml Essigsäure und 2 ml Aethylacetat wird bei 50 Psi Wasserstoff 4 Stunden hydriert. Der Katalysator wird abfiltriert, und das rohe, leicht orange gefärbte Produkt wird nach Konzentrieren durch flash-Chromatographie über Silikagel gereinigt, wobei als Eluiermittel Hexan:Aethylacetat = 2:1 bis 1:1 verwendet wird. Man erhält so nach Zerreiben mit Aether das N,15,16,17,18,19,28,29-Oktahydro-1-desoxy-15-desoxo-1,15-oxy-8-O-pivaloyl-3-[2-methyl--4-(2,4,6-trimethylbenzyl)-piperazin-1-yl]-rifamycin vom Smp. 140-142°.
NMR (300 MHz, $CD_3OD$): 5,76 (s, 1H); 2,81 (br. d, 2H) und 2,88 (br. t, 2H).

Beispiel 21:

In analoger Weise, z.B. wie in Beispiel 1 bei der Herstellung des Ausgangsmaterials beschrieben, erhält man ausgehend von 1,8-Di-O-pivaloyl-3-[4-(4-penten-1-yl)-1-piperazinyl]-rifamycin das 8-O-Pivaloyl-1-desoxy-15-desoxo-1,15-oxy-3-[4-(4-penten-1-yl)-1-piperazinyl]-rifamycin der Formel I worin die Strukturelemente $-A_1-A_2$, $-A_3-A_4-$ und $-A_5-A_6-$ jeweils Vinylen bedeuten, X für >N- steht, alk Methylen bedeutet, $R_1$ Pivaloyl ist, $R_2$ Acetyl ist, $R_3$ und $R_4$ gemeinsam für eine Bindung stehen, $R_5$ 3-Butenyl ist und $R_7$ Wasserstoff ist. Die Verbindung kristallisiert aus Methanol in Plättchen, die bei 140° schmelzen.
$C_{51}H_{69}N_3O_{12}$; MG: 915 (gefunden: MS, DCI).
$^1H$-NMR.-Spektrum (360 MHz, DMSO-$d_6$): Signale der olefinischen Seitenkette in Multipletts, zentriert bei: 1,58 (2H), 2,09 (2H), 2,48 (2H) sowie 4,95 (zusammen mit d von H-25, zusammen 2H), 5,04 (1H) und 6,86 (1H) ppm.

Beispiel 22:

In analoger Weise, z.B. wie in einem der vorstehenden Beispiele beschrieben, kann man folgende Verbindungen herstellen:
8-O-Pivaloyl-1-desoxy-15-desoxo-1,15-oxy-3-[4-(2,3-dimethylbenzyl)-piperazin-1-yl]-rifamycin,
8-O-Pivaloyl-1-desoxy-15-desoxo-1,15-oxy-3-[4-(2,6-dimethylbenzyl)-piperazin-1-yl]-rifamycin,
8-O-Pivaloyl-1-desoxy-15-desoxo-1,15-oxy-3-[4-[2-(2,4,6-trimethylphenyl)-äthyl]-piperazin-1-yl]-rifamycin,
8-O-Pivaloyl-1-desoxy-15-desoxo-1,15-oxy-3-[4-[1-(2,4,6-trimethylphenyl)-äthyl]-piperazin-1-yl]-rifamycin,
8-O-Pivaloyl-1-desoxy-15-desoxo-1,15-oxy-[4-(2-Methoxybenzyl)-piperazin-1-yl]-rifamycin,
8-O-Pivaloyl-1-desoxy-15-desoxo-1,15-oxy-[4-(2,4,6-trimethylphenyl)-piperazin-1-yl]-rifamycin.

Beispiel 23:

Kapseln, enthaltend 250 mg als Wirkstoff z.B. die Verbindung der Formel I, worin die Strukturelemente $-A_1-A_2-$, $-A_3-A_4-$ und $-A_5-A_6-$jeweils Ethylen bedeuten, X für >N- steht, alk Methylen bedeutet, $R_1$ Pivaloyl bedeutet, $R_2$ Acetyl bedeutet, $R_3$ und $R_4$ gemeinsam für eine Bindung stehen, $R_5$ 2,4,6-Trimethylphenyl bedeutet und $R_7$ Wasserstoff ist, können wie folgt hergestellt werden:

Zusammensetzung (für 1000 Kapseln):

| | | |
|---|---|---|
| Wirkstoff | | 250,0 g |
| Maisstärke | | 50,0 g |
| Polyvinylpyrrolidon | | 15,0 g |
| Magnesiumstearat | | 5,0 g |
| Ethanol | q.s. | |

Der Wirkstoff und die Maisstärke werden vermischt und mit einer Lösung des Polyvinylpyrrolidons in 50 g Ethanol befeuchtet. Die feuchte Masse wird durch ein Sieb mit einer Maschenweite von 3 mm gepresst und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse 0 abgefüllt.

In analoger Weise kann man auch die übrigen, gemäss Beispielen 1 bis 22 hergestellten Verbindungen als

Wirkungskomponente verwenden.

**Patentansprüche**

1. Verbindungen der Formel

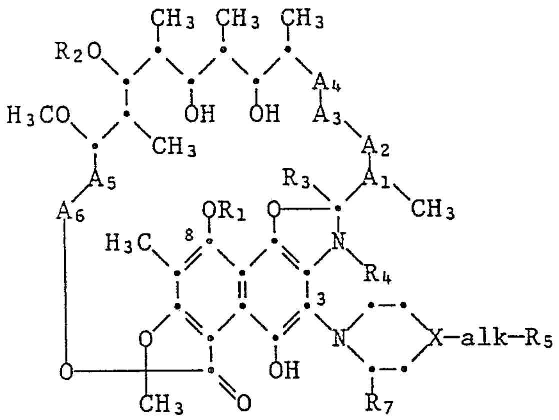

(I)

und ihre Salze, worin die Strukturelemente $-A_1-A_2-$, $-A_3-A_4-$ und $-A_5-A_6-$jeweils Ethylen oder Vinylen oder die Elemente $-A_1-A_2-$ und $-A_3-A_4-$jeweils Ethylen und $-A_5-A_6-$ Vinylen bedeuten, X für $>CR_6-$ oder $^-N-$ und $R_6$ für Alkyl oder Wasserstoff steht, alk einen aliphatischen Kohlenwasserstoffrest oder eine Bindung bedeutet, $R_1$ Wasserstoff oder Acyl bedeutet, $R_2$ Wasserstoff oder Acetyl bedeutet, $R_3$ und $R_4$ gemeinsam für eine Bindung stehen oder jeweils Wasserstoff bedeuten, $R_5$ Wasserstoff, einen cycloaliphatischen Kohlenwasserstoffrest, Aryl oder Heteroaryl bedeutet und $R_7$ Wasserstoff oder Alkyl bedeutet, mit der Massgabe, dass wenn $-A_1-A_2-$, $-A_3-A_4-$ und $-A_5-A_6-$ jeweils Vinylen bedeuten, X für $>N-$ steht, $R_1$ Wasserstoff oder Trialkylacetyl bedeutet, $R_2$ Wasserstoff oder Acetyl bedeutet, $R_3$ und $R_4$ gemeinsam für eine Bindung stehen und alk Methylen bedeutet, $R_5$ von 2,6-Dimethyl-4-alkyl-phenyl verschieden ist.

2. Verbindung gemäss Anspruch 1 der Formel I und ihre Salze, worin $-A_1-A_2-$, $-A_3-A_4-$, $-A_5-A_6-$, $R_2$, $R_3$ und $R_4$ die angegebenen Bedeutungen haben, X für $>CH-$ oder $>N-$ steht, alk Alkylen bedeutet, $R_1$ Wasserstoff oder Acyl bedeutet und $R_5$ Wasserstoff, Cycloalkyl oder Aryl bedeutet und $R_7$ Wasserstoff bedeutet.

3. Verbindung gemäss Anspruch 1 der Formel I und ihre Salze, worin $-A_1-A_2-$, $-A_3-A_4-$, $-A_5-A_6-$, $R_2$, $R_3$ und $R_4$ die angegebenen Bedeutungen haben, X für $>C(R_6)$ oder $>N-$ und $R_6$ für Wasserstoff oder Alkyl steht, alk Alkylen, Alkenylen oder Alkinylen bedeutet, wobei die Mehrfachbindung in höherer als der $\alpha$-Stellung zum Piperazinstickstoff ($X = >N-$) lokalisiert ist, $R_1$ gegebenenfalls durch Halogen oder durch Phenyl substituiertes Alkanoyl, Benzoyl, Naphthoyl oder monocyclisches, 5- bis 6-gliedriges Monoaza-, Monooxa- oder Monothiaaroyl, Alkoxycarbonyl oder gegebenenfalls durch Alkyl mono- oder disubstituiertes Aminocarbonyl bedeutet, $R_5$ Wasserstoff, Cycloalkyl, Cycloalkenyl, Cycloalkinyl, Phenyl, Biphenylyl, Naphthyl, monocyclisches, 5- oder 6-gliedriges Monoazo-, Monooxa- oder Monothiaaryl bedeutet, und $R_7$ Wasserstoff oder Alkyl bedeutet, wobei die aromatischen Reste unabhängig voneinander jeweils unsubstituiert oder ein- oder mehrfach durch Halogen, Alkyl, Alkoxy, Hydroxy, Alkanoyloxy, Trifluormethyl und/oder Nitro substituiert sind.

4. Verbindung gemäss Anspruch 1 der Formel I und ihre Salze, worin $-A_1-A_2-$, $-A_3-A_4-$, $-A_5-A_6-$, $R_2$, $R_3$ und $R_4$ die angegebenen Bedeutungen haben, X für $>C(R_6)$ oder $>N-$ und $R_6$ für Wasserstoff oder $C_1$-$C_7$-Alkyl steht, alk $C_1$-$C_{12}$-Alkylen, $C_3$-$C_7$-Alkenylen oder $C_3$-$C_7$-Alkinylen bedeutet, wobei die Mehrfachbindung in höherer als der $\alpha$-Stellung zum Piperazinstickstoffatom lokalisiert ist, $R_1$ gegebenenfalls durch Halogen oder durch Phenyl substituiertes $C_2$-$C_8$-Alkanoyl, Benzoyl, Naphthoyl oder monocyclisches, 5- bis 6-gliedriges Monoaza-, Monooxa- oder Monothiaaroyl, $C_1$-$C_7$-Alkoxycarbonyl oder gegebenenfalls durch $C_1$-$C_7$-Alkyl mono-oder disubstituiertes Aminocarbonyl bedeutet, $R_5$ Wasserstoff, $C_3$-$C_7$-Cycloalkyl, $C_3$-$C_7$-Cycloalkenyl, $C_3$-$C_7$-Cycloalkinyl, Phenyl, Biphenylyl, Naphthyl, Pyrrolyl, N-$C_1$-$C_7$-Alkyl-pyrrolyl, Pyridyl, 1-Oxido-pyridyl, Furyl oder Thienyl bedeutet, und $R_7$ Wasserstoff oder $C_1$-$C_7$-Alkyl bedeutet, wobei die aromatischen Reste unabhängig voneinander jeweils unsubstituiert oder ein- oder mehrfach durch Halogen, $C_1$-$C_7$-Alkyl, $C_1$-$C_7$-Alkoxy, Hydroxy, $C_2$-$C_8$-Alkanoyloxy, Trifluormethyl und/oder Nitro substituiert sind.

5. Verbindung gemäss Anspruch 1 der Formel I und ihre Salze, worin $-A_1-A_2-$, $-A_3-A_4-$, $-A_5-A_6-$, $R_2$, $R_3$ und $R_4$ die angegebenen Bedeutungen haben, X für $>CH-$ oder $>N-$ steht, alk $C_1$-$C_7$-Alkylen bedeutet, $R_1$ gegebenenfalls durch Halogen oder durch gegebenenfalls Halogen, $C_1$-$C_7$-Alkyl, $C_1$-$C_7$-Alkoxy, Hydroxy, $C_2$-$C_8$-Alkanoyloxy, Trifluormethyl, und/oder Nitro aufweisendes Phenyl substituiertes $C_2$-$C_8$-Alkanoyl, gegebenenfalls durch Halogen, $C_1$-$C_7$-Alkyl, $C_1$-$C_7$-Alkoxy, Hydroxy, $C_2$-$C_8$-Alkanoyloxy,

Trifluormethyl, und/oder Nitro substituiertes Benzoyl, Naphthoyl oder monocyclisches, 5- bis 6-gliedriges Monoaza-, Monooxa- oder Monothiaaroyl, $C_1$-$C_7$-Alkoxycarbonyl oder gegebenenfalls durch $C_1$-$C_7$-Alkyl mono- oder disubstituiertes Aminocarbonyl bedeutet, $R_5$ Wasserstoff, $C_3$-$C_7$-Cycloalkyl oder unsubstituiertes oder ein- oder mehrfach durch Halogen, $C_1$-$C_7$-Alkyl, $C_1$-$C_7$-Alkoxy, Hydroxy, $C_2$-$C_8$-Alkanoyloxy, Trifluormethyl und/oder Nitro substituiertes Phenyl, Biphenylyl oder Naphthyl bedeutet und $R_7$ Wasserstoff ist.

6. Verbindung gemäss Anspruch 1 der Formel I und ihre Salze, worin $-A_1-A_2-$, $-A_3-A_4-$, $-A_5-A_6-$, $R_2$, $R_3$ und $R_4$ die angegebenen Bedeutungen haben, X für $>C(R_6)$ oder $>N-$ und $R_6$ für Wasserstoff oder $C_1$-$C_7$-Akyl steht, alk $C_1$-$C_{12}$-Alkylen, wie $C_1$-$C_7$-Alkylen, $C_3$-$C_7$-Alkenylen oder $C_3$-$C_7$-Alkinylen bedeutet, wobei die Mehrfachbindung in höherer als der $\alpha$-Stellung zum Piperazinstickstoffatom lokalisiert ist, $R_1$ Wasserstoff oder $C_3$-$C_6$-Alkanoyl bedeutet, $R_5$ Wasserstoff, $C_3$-$C_7$-Cycloalkyl, $C_3$-$C_7$-Cycloalkenyl oder unsubstituiertes oder durch Halogen, $C_1$-$C_7$-Alkyl, $C_1$-$C_7$-Alkoxy und/oder Trifluormethyl substituiertes Phenyl, Biphenylyl, Naphthyl, Thienyl, Furyl oder Pyridyl bedeutet und $R_7$ Wasserstoff oder $C_1$-$C_7$-Alkyl bedeutet.

7. Verbindung gemäss Anspruch 1 der Formel I und ihre Salze, worin $-A_1-A_2-$, $-A_3-A_4-$, $-A_5-A_6-$, $R_3$ und $R_4$ die angegebenen Bedeutungen haben, X für $>CH$ oder $>N-$ steht, $R_1$ $C_3$-$C_6$-Alkanoyl, insbesondere Pivaloyl, bedeutet, $R_2$ Acetyl ist, einerseits alk $C_3$-$C_7$-Alkylen, wie 2-Methyl-1,3-propylen, bedeutet und $R_5$ Wasserstoff ist oder andererseits alk $C_1$-$C_4$-Alkylen, insbesondere Methylen, bedeutet, $R_5$ $C_3$-$C_6$-Cycloalkyl, wie Cyclohexyl, $C_3$-$C_6$-Cycloalkenyl, wie Cyclohex-3-en-1-yl, unsubstituiertes oder durch $C_1$-$C_4$-Alkyl, insbesondere Methyl, substituiertes Phenyl, wie 2,5-Dimethyl- oder 2,4,6-Trimethylphenyl, Biphenylyl, wie 4-Biphenylyl, Naphthyl, wie 2-Naphthyl, oder Thienyl, wie 2-Thienyl, bedeutet und $R_7$ Wasserstoff oder $C_1$-$C_4$-Alkyl, wie Methyl, bedeutet.

8. Verbindung gemäss Anspruch 1 der Formel I und ihre Salze, worin $-A_1-A_2-$, $-A_3-A_4-$, $-A_5-A_6-$, $R_3$ und $R_4$ die angegebenen Bedeutungen haben, $R_1$ Wasserstoff oder $C_2$-$C_8$-Alkanoyl, insbesondere verzweigtes $C_3$-$C_6$-Alkanoyl, bedeutet, $R_2$ Acetyl ist, X für $>N-$ steht, alk $C_1$-$C_7$-Alkylen, insbesondere $C_1$-$C_4$-Alkylen, bedeutet, $R_5$ Wasserstoff, $C_3$-$C_7$-Cycloalkyl, gegebenenfalls ein- oder mehrfach durch $C_1$-$C_4$-Alkyl oder Halogen substituiertes Phenyl, Biphenylyl oder Naphthyl bedeutet und $R_7$ Wasserstoff ist.

9. Verbindung gemäss Anspruch 1 der Formel I und ihre Salze, worin die Strukturelemente $-A_1-A_2-$, $-A_3-A_4-$ und $-A_5-A_6-$ die angegebenen Bedeutungen haben, $R_1$ verzweigtes $C_3$-$C_6$-Alkanoyl, wie Pivaloyl, bedeutet, $R_2$ Acetyl ist, $R_3$ und $R_4$ die angegebenen Bedeutungen haben, X für $>N-$ steht, alk $C_1$-$C_4$-Alkylen, wie Methylen, bedeutet, $R_5$ $C_3$-$C_7$-Cycloalkyl, wie Cyclohexyl, $C_3$-$C_7$-Cycloalkenyl, wie Cyclohex-3-en-1-yl, oder durch $C_1$-$C_4$-Alkyl, wie Methyl, ein- oder mehrfach, wie zwei-oder dreifach, substituiertes Phenyl, wie 2-Methylphenyl, 2,3-, 2,5- oder 2,6-Dimethylphenyl oder 2,4,6-Trimethylphenyl, bedeutet und $R_7$ einerseits Wasserstoff, andererseits $C_1$-$C_4$-Alkyl, wie Methyl, bedeutet.

10. Verbindung gemäss Anspruch 1 der Formel I und ihre Salze, worin die Strukturelemente $-A_1-A_2-$, $-A_3-A_4-$ und $-A_5-A_6-$, die Variablen $R_3$ und $R_4$ die angegebenen Bedeutungen haben, alk $C_1$-$C_4$-Alkylen, wie Methylen, 2,3-Propylen oder 2-Methyl-1,3-propylen, bedeutet, $R_1$ Wasserstoff oder verzweigtes $C_3$-$C_6$-Alkanoyl, wie Piovaloyl bedeutet, $R_2$ Acetyl ist, X für $>N-$ steht, $R_5$ Wasserstoff, $C_3$-$C_6$-Cycloalkyl, wie Cyclohexyl, Phenyl oder 2,4,6-Tri-$C_1$-$C_4$-alkyl-phenyl, wie 2,4,6-Trimethylphenyl, bedeutet und $R_7$ Wasserstoff ist.

11. Verbindung gemäss Anspruch 1 der Formel I und ihre Salze, worin die Strukturelemente $-A_1-A_2-$, $-A_3-A_4-$ und $-A_5-A_6-$ die angegebenen Bedeutungen haben, X für $>N-$ steht, $R_2$ Wasserstoff oder Acetyl bedeutet, $R_3$ und $R_4$ gemeinsam für eine Bindung stehen, und einerseits alk $C_1$-$C_4$-Alkylen, in erster Linie 2-Methyl-1,3-propylen, bedeutet und $R_5$ Wasserstoff ist oder andererseits alk Methylen ist und $R_5$ 2,4,6-Tri-$C_1$-$C_4$-alkyl-phenyl, in erster Linie 2,4,6-Trimethylphenyl, bedeutet und $R_7$ jeweils Wasserstoff ist.

12. Verbindung gemäss Anspruch 1 der Formel I und ihre Salze, worin die Strukturelemente $-A_1-A_2-$, $-A_3-A_4-$ und $-A_5-A_6-$ die angegebenen Bedeutungen haben, X für $>N-$ steht, $R_2$ Acetyl bedeutet, $R_3$ und $R_4$ gemeinsam für eine Bindung oder jeweils für Wasserstoff stehen, und einerseits alk $C_1$-$C_4$-Alkylen, in erster Linie 2-Methyl-1,3-propylen, bedeutet und $R_5$ Wasserstoff ist oder andererseits alk Methylen ist und $R_5$ 2,4,6-Tri-$C_1$-$C_4$-alkyl-phenyl, in erster Linie 2,4,6-Trimethylphenyl, bedeutet und $R_7$ jeweils $C_1$-$C_4$-Alkyl, wie Methyl, ist.

13. Verbindung gemäss Anspruch 1 der Formel I und ihre Salze, worin die Strukturelemente $-A_1-A_2-$, $-A_3-A_4-$ und $-A_5-A_6-$ jeweils Vinylen oder die Elemente $-A_1-A_2-A_3-A_4-$ Buta-1,3-dien-1,4-diyl und $-A_5-A_6-$ Ethylen bedeuten, X für $>CH-$ steht, alk $C_1$-$C_4$-Alkylen, insbesondere Methylen oder 2,3-Propylen, bedeutet, $R_1$ verzweigtes $C_3$-$C_6$-Alkanoyl, insbesondere Pivaloyl, bedeutet, $R_2$ Acetyl ist, $R_3$ und $R_4$ gemeinsam für eine Bindung stehen und $R_5$ Wasserstoff, ferner Cyclohexyl oder Phenyl bedeutet und $R_7$ Wasserstoff ist.

14. Verbindung gemäss Anspruch 1 der Formel I und ihre Salze, worin die Strukturelemente $-A_1-A_2-$, $-A_3-A_4-$ und $-A_5-A_6-$ jeweils Vinylen oder die Elemente $-A_1-A_2-A_3-A_4-$ Buta-1,3-dien-1,4-diyl und $-A_5-A_6-$ Ethylen bedeuten, X für $>N-$ steht, $R_1$ Wasserstoff oder verzweigtes $C_3$-$C_6$-Alkanoyl, insbesondere Pivaloyl, bedeutet, $R_2$ Acetyl ist, $R_3$ und $R_4$ gemeinsam für eine Bindung stehen, alk $C_1$-$C_4$-Alkylen, insbesondere 2-Methyl-1,3-propyl, bedeutet und $R_5$ Wasserstoff ist oder alk $C_1$-$C_4$-Alkylen, insbesondere Methylen, bedeutet und $R_5$ 2,4,6-Tri-$C_1$-$C_4$-alkylphenyl, insbesondere 2,4,6-Trimethylphenyl, bedeutet

und $R_7$ Wasserstoff ist.

15. Verbindung gemäss Anspruch 1 der Formel I und ihre Salze, worin X für > N- steht, -$A_1$-$A_2$- und -$A_3$-$A_4$- jeweils Ethylen und $A_5$-$A_6$- Vinylen oder Ethylen bedeuten, $R_1$ verzweigtes $C_3$-$C_6$-Alkanoyl, insbesondere Pivaloyl, bedeutet, $R_2$ Acetyl ist, $R_3$ und $R_4$ gemeinsam für eine Bindung oder jeweils für Wasserstoff stehen, einerseits alk $C_1$-$C_4$-Alkylen, insbesondere Methylen, bedeutet und $R_5$ 2,4,6-Trimethylphenyl bedeutet oder andererseits alk $C_1$-$C_4$-Alkylen, insbesondere 2-Methyl-1,3-propylen, bedeutet und $R_5$ Wasserstoff bedeutet und $R_7$ Wasserstoff ist.

16. Verbindung gemäss Anspruch 1 der Formel I und ihre Salze, worin -$A_1$-$A_2$-, -$A_3$-$A_4$- und -$A_5$-$A_6$- jeweils Ethylen bedeuten, X für > N- steht, $R_1$ Pivaloyl bedeutet, $R_2$ Acetyl ist, $R_3$ und $R_4$ gemeinsam für eine Bindung oder jeweils Wasserstoff bedeuten, alk-$R_5$ 2,4,6-Trimethylbenzyl bedeutet und $R_7$ Wasserstoff ist.

17. 16,17,18,19-Tetrahydro-8-O-pivaloyl-1-desoxy-15-desoxo-1,15-oxy-3-[4-(2,4,6-trimethylbenzyl)-piperazin-1-yl]-rifamycin oder ein Salz davon.

18. 16,17,18,19,28,29-Hexahydro-8-O-pivaloyl-1-desoxy-15-desoxo-1,15-oxy-3-[4-(2,4,6-trimethylbenzyl)-piperazin-1-yl]-rifamycin oder ein Salz davon.

19. N,15,16,17,18,19,28,29-Oktahydro-8-O-pivaloyl-1-desoxy-15-desoxo-1,15-oxy-3-[4-(2,4,6-trimethylbenzyl)-piperazin-1-yl]-rifamycin oder ein Salz davon.

20. 8-O-Pivaloyl-1-desoxy-15-desoxo-1,15-oxy-3-[4-(1-naphthyl-methyl)-piperazin-1-yl]-rifamycin oder ein Salz davon.

21. 8-O-Pivaloyl-1-desoxy-15-desoxo-1,15-oxy-3-(4-isobutyl-1-piperazin-yl)-rifamycin oder ein Salz davon.

22. N,15,16,17,18,19,28,29-Oktahydro-8-O-pivaloyl-1-desoxy-15-desoxo-1,15-oxy -3-(4-isobutyl-1-piperazinyl)-rifamycin oder ein Salz davon.

23. 8-O-Pivaloyl-1-desoxy-15-desoxo-1,15-oxy-3-[4-(4-phenylbenzyl)-1-piperazinyl]-rifamycin oder ein Salz davon.

24. N,15,16,17,18,19-Hexahydro-8-O-pivaloyl-1-desoxy-15-desoxo-1,15-oxy-3-[4-(2,4,6-trimethylbenzyl)-1-piperazinyl]-rifamycin oder ein Salz davon.

25. N,15-Dihydro-8-O-pivaloyl-1-desoxy-15-desoxo-1,15-oxy-3-[4-(2,4,6-trimethylbenzyl)-1-piperazinyl]-rifamycin oder ein Salz davon.

26. 8-O-Pivaloyl-1-desoxy-15-desoxo-1,15-oxy-3-[4-(2,5-dimethylbenzyl)-1-piperazinyl]-rifamycin oder ein Salz davon.

27. 8-O-Pivaloyl-1-desoxy-15-desoxo-1,15-oxy-3-(4-benzyl-1-piperazinyl)-rifamycin oder ein Salz davon.

28. 8-O-Pivaloyl-1-desoxy-15-desoxo-1,15-oxy-3-(4-cyclohexylmethyl-1-piperazinyl)-rifamycin oder ein Salz davon.

29. 8-O-Pivaloyl-1-desoxy-15-desoxo-1,15-oxy-3-[4-(cyclohex-3-en-1-yl-methyl)-1-piperazinyl]-rifamycin oder ein Salz davon.

30. 8-O-Pivaloyl-1-desoxy-15-desoxo-1,15-oxy-3-[2-methyl-4-(2,4,6-trimethylbenzyl)-piperazin-1-yl]-rifamycin oder ein Salz davon.

31. N,15,16,17,18,19,28,29-Oktahydro-1-desoxy-15-desoxo-1,15-oxy-8-O-pivaloyl-3-[2-methyl-4-(2,4,6-trimethylbenzyl)-piperazin-1-yl]-rifamycin oder ein Salz davon.

32. Verbindung gemäss einem der Ansprüche 1-31 in Form eines pharmazeutisch verwendbaren Salzes.

33. Verbindung gemäss einem der Ansprüche 1-31 in Form eines Ascorbinsäuresalzes.

34. Verbindung gemäss einem der Ansprüche 1-31 in Form des (16S)-Isomeren.

35. Verbindung gemäss einem der Ansprüche 1-31 in Form des (16R)-Isomeren.

36. Verbindung gemäss einem der Ansprüche 1-35 zur Anwendung in einem Verfahren zur therapeutischen Behandlung des menschlichen und tierischen Körpers.

37. Pharmazeutische Präparate enthaltend eine Verbindung gemäss einem der Ansprüche 1-36 oder ein pharmazeutisch verwendbares Salz davon zusammen mit üblichen Hilfs- und Zusatzstoffen.

38. Verwendung einer Verbindung gemäss einem der Ansprüche 1-36 oder eines pharmazeutisch verwendbaren Salzes davon zur Herstellung von pharmazeutischen Präparaten zur Behandlung von Hyperlipidämie und Arteriosklerose.

39. Verfahren zur Herstellung von Verbindungen gemäss einem der Ansprüche 1-36, dadurch gekennzeichnet, dass man,

a) zur Herstellung von Verbindungen der Formel I und ihrer Salze, worin X für > N- steht, eine Verbindung der Formel

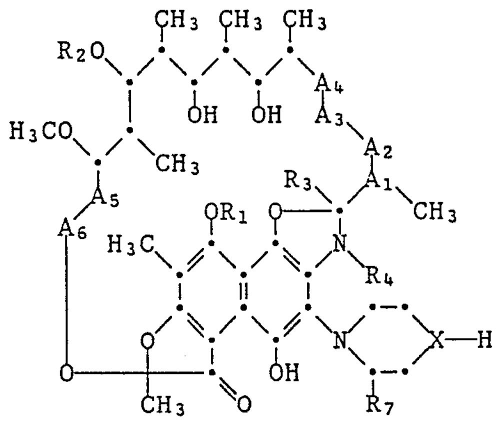

(IIa)

oder ein Salz davon, worin X für > N- steht, mit einer Verbindung der Formel
$Z-alk-R_5$ (IIb)
umsetzt, worin Z reaktionsfähiges verestertes Hydroxy bedeutet, oder,
b) zur Herstellung von Verbindungen der Formel I und ihrer Salze, worin $R_2$ Acetyl ist und $R_3$ und $R_4$ gemeinsam für eine Bindung stehen, eine Verbindung der Formel

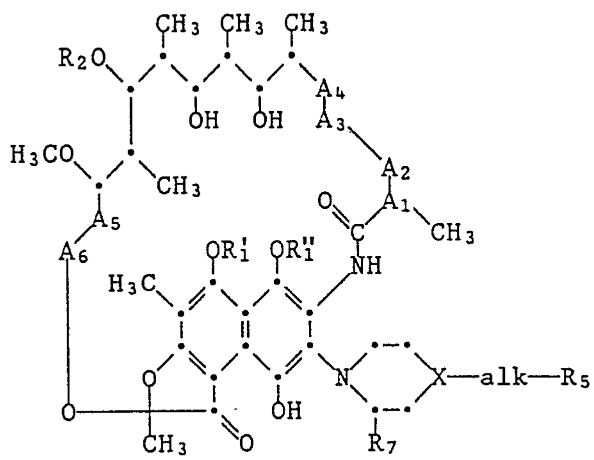

(III) ,

worin R Acyl bedeutet und R Wasserstoff oder Acyl bedeutet, cyclisiert, oder
c) eine Verbindung der Formel

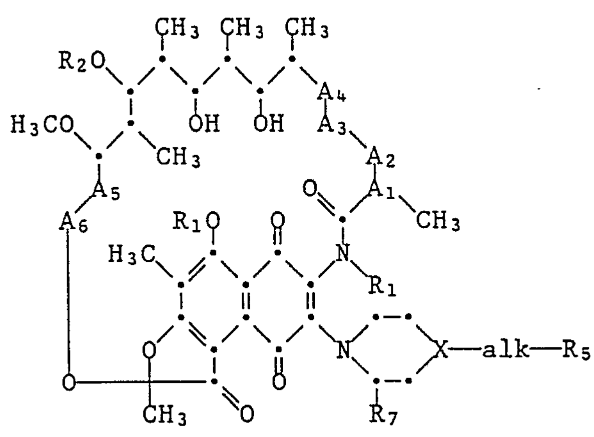

(IV)

cyclisiert,
und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt und, wenn erwünscht, ein verfahrensgemäss erhältliches Gemisch von Isomeren auftrennt.

**Patentansprüche für folgende Vertragsstaaten: ES, GR**

1. Verfahren zur Herstellung von Verbindungen der Formel

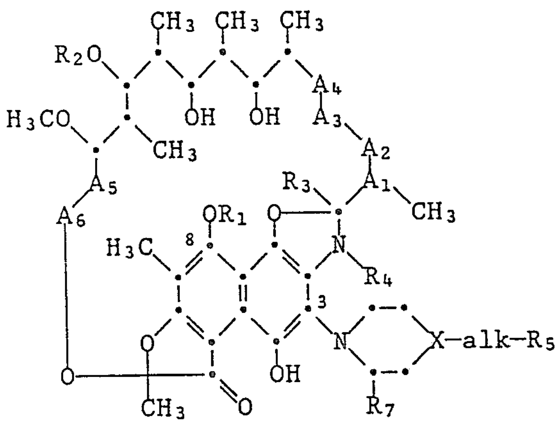

(I)

und ihre Salze, worin die Strukturelemente $-A_1-A_2-$, $-A_3-A_4-$ und $-A_5-A_6-$jeweils Ethylen oder Vinylen oder die Elemente $-A_1-A_2-$ und $-A_3-A_4-$jeweils Ethylen und $-A_5-A_6-$ Vinylen bedeuten, X für $>CR_6-$ oder $^-$N- und $R_6$ für Alkyl oder Wasserstoff steht, alk einen aliphatischen Kohlenwasserstoffrest oder eine Bindung bedeutet, $R_1$ Wasserstoff oder Acyl bedeutet, $R_2$ bedeutet, $R_2$ Wasserstoff oder Acetyl bedeutet, $R_3$ und $R_4$ gemeinsam für eine Bindung stehen oder jeweils Wasserstoff bedeuten, $R_5$ Wasserstoff, einen cycloaliphatischen Kohlenwasserstoffrest, Aryl oder Heteroaryl bedeutet und $R_7$ Wasserstoff oder Alkyl bedeutet, mit der Massgabe, dass wenn $-A_1-A_2-$, $-A_3-A_4-$ und $-A_5-A_6-$ jeweils Vinylen bedeuten, X für $>N-$ steht, $R_1$ Wasserstoff oder Trialkylacetyl bedeutet, $R_2$ Wasserstoff oder Acetyl bedeutet, $R_3$ und $R_4$ gemeinsam für eine Bindung stehen und alk Methylen bedeutet, $R_5$ von 2,6-Dimethyl-4-alkyl-phenyl verschieden ist, dadurch gekennzeichnet, dass man,

a) zur Herstellung von Verbindungen der Formel I und ihre Salze, worin X für $>N-$ steht, eine Verbindung der Formel

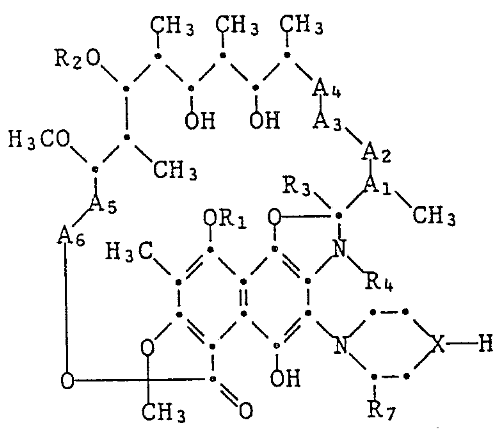

(IIa)

oder ein Salz davon, worin X für $>N-$ steht, mit einer Verbindung der Formel

$Z-alk-R_5$ (IIb)

umsetzt, worin Z reaktionsfähiges verestertes Hydroxy bedeutet, oder,

b) zur Herstellung von Verbindungen der Formel I und ihrer Salze, worin $R_2$ Acetyl ist und $R_3$ und $R_4$ gemeinsam für eine Bindung stehen, eine Verbindung der Formel

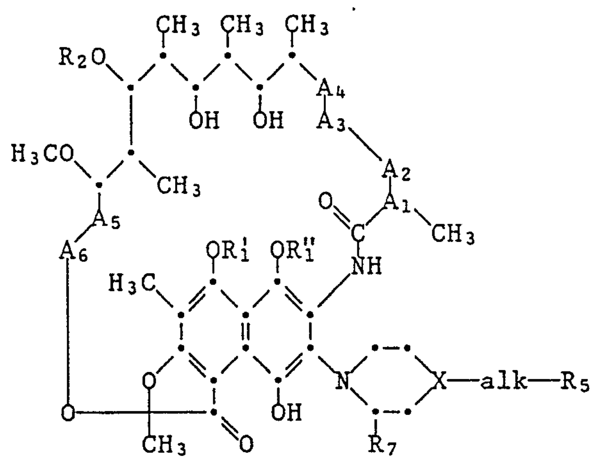

(III) ,

worin R Acyl bedeutet und R Wasserstoff oder Acyl bedeutet, cyclisiert, oder
c) eine Verbindung der Formel

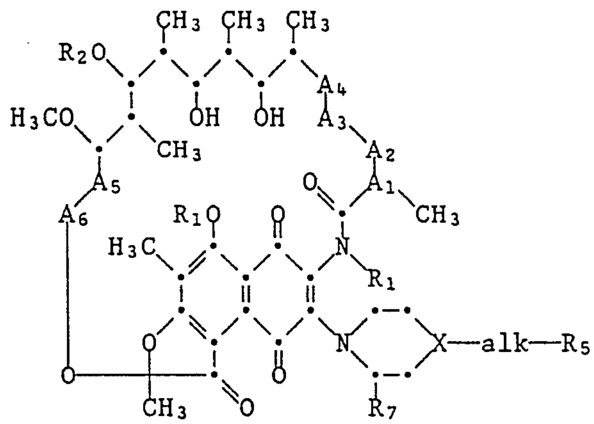

(IV)

cyclisiert,
und gewünschtenfalls eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz und/oder ein verfahrensgemäss erhältliches Salz in die freie Verbindung der Formel I oder in ein anderes Salz überführt und, wenn erwünscht, ein verfahrensgemäss erhältliches Gemisch von Isomeren auftrennt.

2. Verfahren gemäss Anspruch 1 zur Herstellung von Verbindungen der Formel I und ihrer Salze, worin $-A_1-A_2-$, $-A_3-A_4-$, $-A_5-A_6-$, $R_2$, $R_3$ und $R_4$ die angegebenen Bedeutungen haben, X für $>$CH- oder $^-$N- steht, alk Alkylen bedeutet, $R_1$ Wasserstoff oder Acyl bedeutet und $R_5$ Wasserstoff, Cycloalkyl oder Aryl bedeutet und $R_7$ Wasserstoff bedeutet.

3. Verfahren gemäss Anspruch 1 zur Herstellung von Verbindungen der Formel I und ihrer Salze, worin $-A_1-A_2-$, $-A_3-A_4-$, $-A_5-A_6-$, $R_2$, $R_3$ und $R_4$ die angegebenen Bedeutungen haben, X für $>C(R_6)$ oder $^-$N- und $R_6$ für Wasserstoff oder Alkyl steht, alk Alkylen, Alkenylen oder Alkinylen bedeutet, wobei die Mehrfachbindung in höherer als der $\alpha$-Stellung zum Piperazinstickstoff (X = $>$N-) lokalisiert ist, $R_1$ gegebenenfalls durch Halogen oder durch Phenyl substituiertes Alkanoyl, Benzoyl, Naphthoyl oder monocyclisches, 5- bis 6-gliedriges Monoaza-, Monooxa- oder Monothiaaroyl, Alkoxycarbonyl oder gegebenenfalls durch Alkyl mono- oder disubstituiertes Aminocarbonyl bedeutet, $R_5$ Wasserstoff, Cycloalkyl, Cycloalkenyl, Cycloalkinyl, Phenyl, Biphenyl, Naphthyl, monocyclisches, 5- oder 6-gliedriges Monoazo-, Monooxa- oder Monothiaaryl bedeutet, und $R_7$ Wasserstoff oder Alkyl bedeutet, wobei die aromatischen Reste unabhängig voneinander jeweils unsubstituiert oder ein- oder mehrfach durch Halogen, Alkyl, Alkoxy, Hydroxy, Alkanoyloxy, Trifluormethyl und/oder Nitro substituiert sind.

4. Verfahren gemäss Anspruch 1 zur Herstellung von Verbindungen der Formel I und ihrer Salze, worin $-A_1-A_2-$, $-A_3-A_4-$, $-A_5-A_6-$, $R_2$, $R_3$ und $R_4$ die angegebenen Bedeutungen haben, X für $>C(R_6)$ oder $^-$N- und $R_6$ für Wasserstoff oder $C_1$-$C_7$-Alkyl steht, alk $C_1$-$C_{12}$-Alkylen, $C_3$-$C_7$-Alkenylen oder $C_3$-$C_7$-Alkinylen bedeutet, wobei die Mehrfachbindung in höherer als der $\alpha$-Stellung zum Piperazinstickstoffatom lokalisiert ist, $R_1$ gegebenenfalls durch Halogen oder durch Phenyl substituiertes $C_2$-$C_8$-Alkanoyl, Benzoyl, Naphthoyl oder monocyclisches, 5- bis 6-gliedriges Monoaza-, Monooxa- oder Monothiaaroyl, $C_1$-$C_7$-Alkoxycarbonyl oder gegebenenfalls durch $C_1$-$C_7$-Alkyl mono-oder disubstituiertes Aminocarbonyl bedeutet, $R_5$ Wasserstoff, $C_3$-$C_7$-Cycloalkyl, $C_3$-$C_7$-Cycloalkenyl, $C_3$-$C_7$-Cycloalkinyl, Phenyl,

Biphenylyl, Naphthyl, Pyrrolyl, N-$C_1$-$C_7$-Alkyl-pyrrolyl, Pyridyl, 1-Oxido-pyridyl, Furyl oder Thienyl bedeutet, und $R_7$ Wasserstoff oder $C_1$-$C_7$-Alkyl bedeutet, wobei die aromatischen Reste unabhängig voneinander jeweils unsubstituiert oder ein- oder mehrfach durch Halogen, $C_1$-$C_7$-Alkyl, $C_1$-$C_7$-Alkoxy, Hydroxy, $C_2$-$C_8$-Alkanoyloxy, Trifluormethyl und/oder Nitro substituiert sind.

5. Verfahren gemäss Anspruch 1 zur Herstellung von Verbindungen der Formel I und ihrer Salze, worin -$A_1$-$A_2$-, -$A_3$-$A_4$-, -$A_5$-$A_6$-, $R_2$, $R_3$ und $R_4$ die angegebenen Bedeutungen haben, X für > CH- oder -N- steht, alk $C_1$-$C_7$-Alkylen bedeutet, $R_1$ gegebenenfalls durch Halogen oder durch gegebenenfalls Halogen, $C_1$-$C_7$-Alkyl, $C_1$-$C_7$-Alkoxy, Hydroxy, $C_2$-$C_8$-Alkanoyloxy, Trifluormethyl, und/oder Nitro aufweisendes Phenyl substituiertes $C_2$-$C_8$-Alkanoyl, gegebenenfalls durch Halogen, $C_1$-$C_7$-Alkyl, $C_1$-$C_7$-Alkoxy, Hydroxy, $C_2$-$C_8$-Alkanoyloxy, Trifluormethyl, und/oder Nitro substituiertes Benzoyl, Naphthoyl oder monocyclisches, 5- bis 6-gliedriges Monoaza-, Monooxa- oder Monothiaaroyl, $C_1$-$C_7$-Alkoxycarbonyl oder gegebenenfalls durch $C_1$-$C_7$-Alkyl mono- oder disubstituiertes Aminocarbonyl bedeutet, $R_5$ Wasserstoff, $C_3$-$C_7$-Cycloalkyl oder unsubstituiertes oder ein- oder mehrfach durch Halogen, $C_1$-$C_7$-Alkyl, $C_1$-$C_7$-Alkoxy, Hydroxy, $C_2$-$C_8$-Alkanoyloxy, Trifluormethyl und/oder Nitro substituiertes Phenyl, Biphenylyl oder Naphthyl bedeutet und $R_7$ Wasserstoff ist.

6. Verfahren gemäss Anspruch 1 zur Herstellung von Verbindungen der Formel I und ihrer Salze, worin -$A_1$-$A_2$-, -$A_3$-$A_4$-, -$A_5$-$A_6$-, $R_2$, $R_3$ und $R_4$ die angegebenen Bedeutungen haben, X für > C($R_6$) oder -N- und $R_6$ für Wasserstoff oder $C_1$-$C_7$-Alkyl steht, alk $C_1$-$C_{12}$-Alkylen, wie $C_1$-$C_7$-Alkylen, $C_3$-$C_7$-Alkenylen oder $C_3$-$C_7$-Alkinylen bedeutet, wobei die Mehrfachbindung in höherer als der $\alpha$-Stellung zum Piperazinstickstoffatom lokalisiert ist, $R_1$ Wasserstoff oder $C_3$-$C_6$-Alkanoyl bedeutet, $R_5$ Wasserstoff, $C_3$-$C_7$-Cycloalkyl, $C_3$-$C_7$-Cycloalkenyl oder unsubstituiertes oder durch Halogen, $C_1$-$C_7$-Alkyl, $C_1$-$C_7$-Alkyl und/oder Trifluormethyl substituiertes Phenyl, Biphenylyl, Naphthyl, Thienyl, Furyl oder Pyridyl bedeutet und $R_7$ Wasserstoff oder $C_1$-$C_7$-Alkyl bedeutet.

7. Verfahren gemäss Anspruch 1 zur Herstellung von Verbindungen der Formel I und ihre Salze, worin -$A_1$-$A_2$-, -$A_3$-$A_4$-, -$A_5$-$A_6$-, $R_3$ und $R_4$ die angegebenen Bedeutungen haben, X für > CH oder -N- steht, $R_1$ $C_3$-$C_6$-Alkanoyl, insbesondere Pivaloyl, bedeutet, $R_2$ Acetyl ist, einerseits alk $C_3$-$C_7$-Alkylen, wie 2-Methyl-1,3-propylen, bedeutet und $R_5$ Wasserstoff ist oder andererseits alk $C_1$-$C_4$-Alkylen, insbesondere Methylen, bedeutet, $R_5$ $C_3$-$C_6$-Cycloalkyl, wie Cyclohexyl, $C_3$-$C_6$-Cycloalkenyl, wie Cyclohex-3-en-1-yl, unsubstituiertes oder durch $C_1$-$C_4$-Alkyl, insbesondere Methyl, substituiertes Phenyl, wie 2,5-Dimethyl- oder 2,4,6-Trimethylphenyl, Biphenylyl, wie 4-Biphenylyl, Naphthyl, wie 2-Naphthyl, oder Thienyl, wie 2-Thienyl, bedeutet und $R_7$ Wasserstoff oder $C_1$-$C_4$-Alkyl, wie Methyl, bedeutet.

8. Verfahren gemäss Anspruch 1 zur Herstellung von Verbindungen der Formel I und ihre Salze, worin -$A_1$-$A_2$-, -$A_3$-$A_4$-, -$A_5$-$A_6$-, $R_3$ und $R_4$ die angegebenen Bedeutungen haben, $R_1$ Wasserstoff oder $C_2$-$C_8$-Alkanoyl, insbesondere verzweigtes $C_3$-$C_6$-Alkanoyl, bedeutet, $R_2$ Acetyl ist, X für > N- steht, alk $C_1$-$C_7$-Alkylen, insbesondere $C_1$-$C_4$-Alkylen, bedeutet, $R_5$ Wasserstoff, $C_3$-$C_7$-Cycloalkyl, gegebenenfalls ein- oder mehrfach durch $C_1$-$C_4$-Alkyl oder Halogen substituiertes Phenyl, Biphenylyl oder Naphthyl bedeutet und $R_7$ Wasserstoff ist.

9. Verfahren gemäss Anspruch 1 zur Herstellung von Verbindungen der Formel I und ihre Salze, worin die Strukturelemente -$A_1$-$A_2$-, -$A_3$-$A_4$- und -$A_5$-$A_6$- die angegebenen Bedeutungen haben, $R_1$ verzweigtes $C_3$-$C_6$-Alkanoyl, wie Pivaloyl, bedeutet, $R_2$ Acetyl ist, $R_3$ und $R_4$ die angegebenen Bedeutungen haben, X für > N- steht, alk $C_1$-$C_4$-Alkylen, wie Methylen, bedeutet, $R_5$ $C_3$-$C_7$-Cycloalkyl, wie Cyclohexyl, $C_3$-$C_7$-Cycloalkenyl, wie Cyclohex-3-en-1-yl, oder durch $C_1$-$C_4$-Alkyl, wie Methyl, ein- oder mehrfach, wie zwei-oder dreifach, substituiertes Phenyl, wie 2-Methylphenyl, 2,3-, 2,5- oder 2,6-Dimethylphenyl oder 2,4,6-Trimethylphenyl, bedeutet und $R_7$ einerseits Wasserstoff, andererseits $C_1$-$C_4$-Alkyl, wie Methyl, bedeutet.

10. Verfahren gemäss Anspruch 1 zur Herstellung von Verbindungen der Formel I und ihrer Salze, worin die Strukturelemente -$A_1$-$A_2$-, -$A_3$-$A_4$- und -$A_5$-$A_6$-, die Variablen $R_3$ und $R_4$ die angegebenen Bedeutungen haben, alk $C_1$-$C_4$-Alkylen, wie Methylen, 2,3-Propylen oder 2-Methyl-1,3-propylen, bedeutet, $R_1$ Wasserstoff oder verzweigtes $C_3$-$C_6$-Alkanoyl, wie Pivaloyl bedeutet, $R_2$ Acetyl ist, X für N- steht, $R_5$ Wasserstoff, $C_3$-$C_6$-Cycloalkyl, wie Cyclohexyl, Phenyl oder 2,4,6-Tri-$C_1$-$C_4$-alkyl-phenyl, wie 2,4,6-Trimethylphenyl, bedeutet und $R_7$ Wasserstoff ist.

11. Verfahren gemäss Anspruch 1 zur Herstellung von Verbindungen der Formel I und ihrer Salze, worin die Strukturelemente -$A_1$-$A_2$-, -$A_3$-$A_4$- und -$A_5$-$A_6$- die angegebenen Bedeutungen haben, X für > N- steht, $R_2$ Wasserstoff oder Acetyl bedeutet, $R_3$ und $R_4$ gemeinsam für eine Bindung stehen, und einerseits alk $C_1$-$C_4$-Alkylen, in erster Linie 2-Methyl-1,3-propylen, bedeutet und $R_5$ Wasserstoff ist oder andererseits alk Methylen ist und $R_5$, 2,4,6-Tri-$C_1$-$C_4$-alkyl-phenyl, in erster Linie 2,4,6-Trimethylphenyl, bedeutet und $R_7$ jeweils Wasserstoff ist.

12. Verfahren gemäss Anspruch 1 zur Herstellung von Verbindungen der Formel I und ihrer Salze, worin die Strukturelemente -$A_1$-$A_2$-, -$A_3$-$A_4$- und -$A_5$-$A_6$- die angegebenen Bedeutungen haben, X für > N- steht, $R_2$ Acetyl bedeutet, $R_3$ und $R_4$ gemeinsam für eine Bindung oder jeweils für Wasserstoff stehen, und einerseits alk $C_1$-$C_4$-Alkylen, in erster Linie 2-Methyl-1,3-propylen, bedeutet und $R_5$ Wasserstoff ist oder andererseits alk Methylen ist und $R_5$ 2,4,6-Tri-$C_1$-$C_4$-alkyl-phenyl, in erster Linie 2,4,6-Trimethylphenyl, bedeutet und $R_7$ jeweils $C_1$-$C_4$-Alkyl, wie Methyl, ist.

13. Verfahren gemäss Anspruch 1 zur Herstellung von Verbindungen der Formel I und ihre Salze, worin die Strukturelemente -$A_1$-$A_2$-, -$A_3$-$A_4$- und -$A_5$-$A_6$- jeweils Vinylen oder die Elemente -$A_1$-$A_2$-$A_3$-$A_4$-

Buta-1,3-dien-1,4-diyl und $-A_5-A_6-$ Ethylen bedeuten, X für > CH- steht, alk $C_1-C_4$-Alkylen, insbesondere Methylen oder 2,3-Propylen, bedeutet, $R_1$ verzweigtes $C_3-C_6$-Alkanoyl, insbesondere Pivaloyl, bedeutet, $R_2$ Acetyl ist, $R_3$ und $R_4$ gemeinsam für eine Bindung stehen und $R_5$ Wasserstoff, ferner Cyclohexyl oder Phenyl bedeutet und $R_7$ Wasserstoff ist.

14. Verfahren gemäss Anspruch 1 zur Herstellung von Verbindungen der Formel I und ihrer Salze, worin die Strukturelemente $-A_1-A_2-$, $-A_3-A_4-$ und $-A_5-A_6-$ jeweils Vinylen oder die Elemente $-A_1-A_2-A_3-A_4-$ Buta-1,3-dien-1,4-diyl und $-A_5-A_6-$ Ethylen bedeuten, X für > N- steht, $R_1$ Wasserstoff oder verzweigtes $C_3-C_6$-Alkanoyl, insbesondere Pivaloyl, bedeutet, $R_2$ Acetyl ist, $R_3$ und $R_4$ gemeinsam für eine Bindung stehen, alk $C_1-C_4$-Alkylen, insbesondere 2-Methyl-1,3-propyl, bedeutet und $R_5$ Wasserstoff ist oder alk $C_1-C_4$-Alkylen, insbesondere Methylen, bedeutet und $R_5$ 2,4,6-Tri-$C_1-C_4$-alkyl-phenyl, insbesondere 2,4,6-Trimethylphenyl, bedeutet und $R_7$ Wasserstoff ist.

15. Verfahren gemäss Anspruch 1 zur Herstellung von Verbindungen der Formel I und ihrer Salze, worin X für > N- steht, $-A_1-A_2-$ und $-A_3-A_4-$ jeweils Ethylen und $A_5-A_6-$ Vinylen oder Ethylen bedeuten, $R_1$ verzweigtes $C_3-C_6$-Alkanoyl, insbesondere Pivaloyl, bedeutet, $R_2$ Acetyl ist, $R_3$ und $R_4$ gemeinsam für eine Bindung oder jeweils für Wasserstoff stehen, einerseits alk $C_1-C_4$-Alkylen, insbesondere Methylen, bedeutet und $R_5$ 2,4,6-Trimethylphenyl bedeutet oder andererseits alk $C_1-C_4$-Alkylen, insbesondere 2-Methyl-1,3-propylen, bedeutet und $R_5$ Wasserstoff bedeutet und $R_7$ Wasserstoff ist.

16. Verfahren gemäss Anspruch 1 zur Herstellung von Verbindungen der Formel I und ihrer Salze, worin $-A_1-A_2-$, $-A_3-A_4-$ und $-A_5-A_6-$ jeweils Ethylen bedeuten, X für > N- steht, $R_1$ Pivaloyl bedeutet, $R_2$ Acetyl ist, $R_3$ und $R_4$ gemeinsam für eine Bindung oder jeweils Wasserstoff bedeuten, alk-$R_5$ 2,4,6-Trimethylbenzyl bedeutet und $R_7$ Wasserstoff ist.

17. Verfahren gemäss Anspruch 1 zur Herstellung von 16,17,18,19-Tetrahydro-8-O-pivaloyl-1-desoxy-15-desoxo-1,15-oxy-3-[4-(2,4,6-trimethylbenzyl)-piperazin-1-yl]-rifamycin oder eines Salzes davon.

18. Verfahren gemäss Anspruch 1 zur Herstellung von 16,17,18,19,28,29-Hexahydro-8-O-pivaloyl-1-desoxy-15-desoxo-1,15-oxy-3-[4-(2,4,6-trimethylbenzyl)-piperazin-1-yl]-rifamycin oder eines Salzes davon.

19. Verfahren gemäss Anspruch 1 zur Herstellung von N,15,16,17,18,19,28,29-Oktahydro-8-O-pivaloyl-1-desoxy-15-desoxo-1,15-oxy-3-[4-(2,4,6-trimethylbenzyl)-piperazin-1-yl]-rifamycin oder eines Salzes davon.

20. Verfahren gemäss Anspruch 1 zur Herstellung von 8-O-Pivaloyl-1-desoxy-15-desoxo-1,15-oxy--3-[4-(1-naphthyl-methyl)-piperazin-1-yl]-rifamycin oder eines Salzes davon.

21. Verfahren gemäss Anspruch 1 zur Herstellung von 8-O-Pivaloyl-1-desoxy-15-desoxo-1,15-oxy-3-(4-isobutyl-1-piperazinyl)-rifamycin oder eines Salzes davon.

22. Verfahren gemäss Anspruch 1 zur Herstellung von N,15,16,17,18,19,28,29-Oktahydro-8-O-pivaloyl-1-desoxy-15-desoxo-1,15-oxy-3-(4-isobutyl-1-piperazinyl)-rifamycin oder eines Salzes davon.

23. Verfahren gemäss Anspruch 1 zur Herstellung von 8-O-Pivaloyl-1-desoxy-15-desoxo-1,15-oxy--3-[4-(4-phenylbenzyl)-1-piperazinyl]-rifamycin oder eines Salzes davon.

24. Verfahren gemäss Anspruch 1 zur Herstellung von N,15,16,17,18,19-Hexahydro-8-O-pivaloyl-1-desoxy-15-desoxo-1,15-oxy-3-[4-(2,4,6-trimethylbenzyl)-1-piperazinyl]-rifamycin oder eines Salzes davon.

25. Verfahren gemäss Anspruch 1 zur Herstellung von N,15-Dihydro-8-O-pivaloyl-1-desoxy-15-desoxo-1,15-oxy-3-[4-(2,4,6-trimethylbenzyl)-1-piperazinyl]-rifamycin oder eines Salzes davon.

26. Verfahren gemäss Anspruch 1 zur Herstellung von 8-O-Pivaloyl-1-desoxy-15-desoxo-1,15-oxy--3-[4-(2,5-dimethylbenzyl)-1-piperazinyl]-rifamycin oder eines Salzes davon.

27. Verfahren gemäss Anspruch 1 zur Herstellung von 8-O-Pivaloyl-1-desoxy-15-desoxo-1,15-oxy-3-(4-benzyl-1-piperazinyl)-rifamycin oder eines Salzes davon.

28. Verfahren gemäss Anspruch 1 zur Herstellung von 8-O-Pivaloyl-1-desoxy-15-desoxo-1,15-oxy-3-(4-cyclohexylmethyl-1-piperazinyl)-rifamycin oder eines Salzes davon.

29. Verfahren gemäss Anspruch 1 zur Herstellung von 8-O-Pivaloyl-1-desoxy-15-desoxo-1,15-oxy--3-[4-(cyclohex-3-en-1-yl-methyl)-1-piperazinyl]-rifamycin oder eines Salzes davon.

30. Verfahren gemäss Anspruch 1 zur Herstellung von 8-O-Pivaloyl-1-desoxy-15-desoxo-1,15-oxy-3-[2-methyl-4-(2,4,6-trimethylbenzyl)-piperazin-1-yl]-rifamycin oder eines Salzes davon.

31. Verfahren gemäss Anspruch 1 zur Herstellung von N,15,16,17,18,19,28,29-Oktahydro-1-desoxy-15-desoxo-1,15-oxy-8-O-pivaloyl-3-[2-methyl-4-(2,4,6-trimethylbenzyl)-piperazin-1-yl]-rifamycin oder eines Salzes davon.

32. Verfahren gemäss Anspruch 1 zur Herstellung einer Verbindung gemäss einem der Ansprüche 1-31 in Form eines pharmazeutisch verwendbaren Salzes.

33. Verfahren gemäss Anspruch 1 zur Herstellung einer Verbindung gemäss Anspruch 1 in Form eines Ascorbinsäuresalzes.

34. Verfahren gemäss Anspruch 1 zur Herstellung einer Verbindung gemäss Anspruch 1 in Form des (16S)-Isomeren.

35. Verfahren gemäss Anspruch 1 zur Herstellung einer Verbindung gemäss Anspruch 1 in Form des (16R)-Isomeren.

36. Verfahren zur Herstellung von pharmazeutischen Präparaten enthaltend eine Verbindung gemäss einem der Ansprüche 1-35 oder eines pharmazeutisch verwendbaren Salzes davon, dadurch gekennzeichnet, dass man den Wirkstoff zusammen mit üblichen Hilfs- und Zusatzstoffen zu pharmazeutischen Präparaten verarbeitet.

37. Verwendung einer Verbindung gemäss einem der Ansprüche 1-35 oder eines pharmazeutisch verwendbaren Salzes davon zur Herstellung von pharmazeutischen Präparaten zur Behandlung von Hyperlipidämie und Arteriosklerose.

Europäisches Patentamt

# EUROPÄISCHER RECHERCHENBERICHT

Nummer der Anmeldung

EP 89 81 0481

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-8 702 361 (CIBA-GEIGY)<br>* Ansprüche 1,8 *<br>--- | 1,37 | C 07 D 498/18<br>A 61 K 31/495//<br>(C 07 D 498/18<br>C 07 D 323:00<br>C 07 D 307:00<br>C 07 D 263:00 ) |
| Y | CHEMICAL PHARMACEUTICAL BULLETIN, Band 33, Nr. 5, 1985, Seiten 2133-2136, Tokyo, JP; M. TAGUCHI et al.: "Chemical modification of the amide group of rifamycin S"<br>* Formel 8 *<br>--- | 1,37 | |
| P,A | EP-A-0 314 624 (CIBA-GEIGY)<br>* Ansprüche 1,14 *<br>----- | 1,37 | |
| | | | **RECHERCHIERTE SACHGEBIETE (Int. Cl.5)** |
| | | | C 07 D 498/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 12-10-1989 | ALFARO I. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)